(19) Europäisches Patentamt
European Patent Office
Office européen des brevets

(11) Veröffentlichungsnummer: **0 353 667 B1**

(12) # EUROPÄISCHE PATENTSCHRIFT

(45) Veröffentlichungstag der Patentschrift: **16.03.94**

(21) Anmeldenummer: **89114030.3**

(22) Anmeldetag: **29.07.89**

Die Akte enthält technische Angaben, die nach dem Eingang der Anmeldung eingereicht wurden und die nicht in dieser Patentschrift enthalten sind.

(51) Int. Cl.5: **C07D 333/38**, C07D 333/18, C07D 333/08, C07D 333/22, C07D 333/28, C07D 333/44, C07D 409/14, C07D 413/14, C07D 417/14, A01N 43/10, A01N 43/36, //A01N43/40, A01N43/42,A01N43/48, A01N43/50,A01N43/76, A01N43/80,A01N43/78

(54) **Bithienylderivate, Verfahren zu ihrer Herstellung und sie enthaltende Mittel.**

(30) Priorität: **04.08.88 DE 3826493**
**03.03.89 DE 3906811**

(43) Veröffentlichungstag der Anmeldung:
**07.02.90 Patentblatt 90/06**

(45) Bekanntmachung des Hinweises auf die Patenterteilung:
**16.03.94 Patentblatt 94/11**

(84) Benannte Vertragsstaaten:
**CH DE ES FR GB IT LI NL**

(56) Entgegenhaltungen:
**WO-A-86/05949**
**SU-A- 495 314**
**US-A- 3 050 442**
**US-A- 3 086 854**

(73) Patentinhaber: **BASF Aktiengesellschaft**
**Carl-Bosch-Strasse 38**
**D-67063 Ludwigshafen(DE)**

(72) Erfinder: **Kober, Reiner, Dr.**
**Im Schlittweg 20**
**D-6701 Fussgoenheim(DE)**
Erfinder: **Leyendecker, Joachim, Dr.**
**Stahlbuehlring 79**
**D-6802 Ladenburg(DE)**
Erfinder: **Seele, Rainer, Dr.**
**Leiblstrasse 3**
**D-6701 Fussgoenheim(DE)**
Erfinder: **Fischer, Klaus, Dr.**
**Gabelsbergerstrasse 7**
**D-6720 Speyer(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 353 667 B1

JOURNAL OF THE CHEMICAL SOCIETY, Perkin Transaction II, 1972; R.E. ATKINSON et al., Seiten 27-30&NUM;

CHIMIKO-FARMACEUTICESKIJ ZURNAL, Band 7, Nr. 8, Moskau (RU); N.I. MIRYAN et al., Seiten 13-17&NUM;

Erfinder: **Theobald, Hans, Dr.**
**Oueichstrasse 6**
**D-6703 Limburgerhof(DE)**
Erfinder: **Wuerzer, Bruno, Dr.**
**Ruedigerstrasse 13**
**D-6701 Otterstadt(DE)**
Erfinder: **Westphalen, Karl-Otto, Dr.**
**Mausbergweg 58**
**D-6720 Speyer(DE)**
Erfinder: **Meyer, Norbert, Dr.**
**Dossenheimer Weg 22**
**D-6802 Ladenburg(DE)**

## Beschreibung

Die vorliegende Erfindung betrifft Bithienylderivate der allgemeinen Formel Ia,

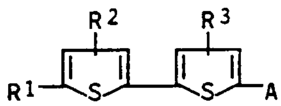

Ia

in der die Substituenten folgende Bedeutung haben:

$R^1$, $R^2$ Wasserstoff, Halogen, Nitro, Formyl, $C_1$-$C_6$-Alkyl, $C_1$-$C_6$-Halogenalkyl, $C_3$-$C_8$-Cycloalkyl, $C_3$-$C_8$-Cycloalkoxy, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_6$-Alkylcarbonyl, welches ein- bis dreifach durch Halogenatome substituiert sein kann, $C_3$-$C_6$-Cycloalkylcarbonyl, welches ein- bis dreifach durch Halogen und/oder $C_1$-$C_4$-Alkyl substituiert sein kann, $C_1$-$C_6$-Alkoxycarbonyl und/oder Phenylcarbonyl;

$R^3$ Wasserstoff, ein Halogenatom, eine $C_1$-$C_4$-Alkylgruppe, eine $C_1$-$C_4$-Alkoxygruppe, eine $C_1$-$C_4$-Alkylthiogruppe, eine $C_1$-$C_4$-Halogenalkylgruppe oder eine $C_1$-$C_4$-Halogenalkoxygruppe;

A ein Rest $-CSNH_2$;
oder ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Substituenten tragen können:

- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein bis fünffach durch Halogen und/oder ein bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder
- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

Außerdem betrifft die Erfindung Verfahren zur Herstellung der Verbindungen Ia sowie herbizide Mittel, welche ein Bithienylderivat der allgemeinen Formel Ia enthalten.

Aus der US-A 3 050 442 sind nematozide Substanzen u.a. der allgemeinen Formel I'

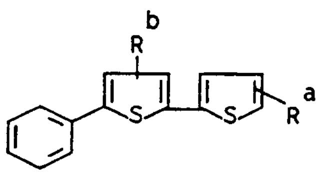

I'

bekannt, in der die Reste beispielsweise folgende Bedeutung haben:
$R^a$ und $R^b$ unabhängig voneinander Wasserstoff, eine Alkylgruppe, eine aliphatische Acylgruppe, ein Halogenatom und/oder eine Nitrogruppe.

Neben der erfindungsgemäßen insektiziden Eigenschaft wird dort auch auf phytotoxische Effekte hingewiesen, die für α-Bithienyl und α-Terthienyl an experimentellen Beispielen belegt sind.

Daneben werden in der WO-A 86/05949 Phenylbithienylderivate I''

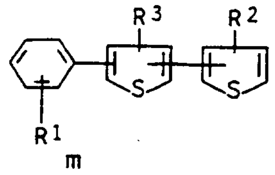

I''

beschrieben, deren allgemeine Definition der Reste und Indices von den Phenyl-bithienylderivate I umfaßt werden. Diese Verbindungen werden als Insektizide und Akarizide empfohlen, wobei ausdrücklich darauf hingewiesen wird, daß bei den applizierten Dosen (50 bis 750 g/ha, vorzugsweise 150 bis 500 g/ha) unter den Versuchsbedingungen keine Probleme mit der Phytotoxizität auftraten.

Die sowjetische Offenlegungsschrift SU 495 314 offenbart unsubstituierte Bithienyle mit herbizider und nematozider Wirkung. Die herbizide Aktivität dieser Verbindungen ist jedoch ausgesprochen schwach.

Der Erfindung lagen neue Bithienylderivate und herbizide Mittel als Aufgabe zugrunde, die bei geringer Aufwandmenge eine hohe Selektivität aufweisen, d.h. unerwünschte Pflanzen gut bekämpfen, ohne dabei die Kulturpflanzen zu schädigen.

Entsprechend dieser Aufgabe wurden die eingangs definierten Verbindungen Ia und herbizide Mittel mit den eingangs definierten Bithienylderivaten Ia gefunden.

Man erhält die Verbindungen Ia, in denen A eine Gruppe $-CSNH_2$ bedeutet beispielsweise dadurch, daß man ein entsprechend substituiertes Bithienylcyanid II in an sich bekannter Weise (Helv. Chim. Acta, 1960, Seite 1522 ff)

a) entweder in Gegenwart einer Base mit Schwefelwasserstoff oder

b) in Gegenwart einer Säure mit einer Thiocarbonsäure III umsetzt.

Journal of the Chemical Society, Perkin Transactions II, 1972, Seiten 27-30, sind die Herstellung sowie spektroskopischen Eigenschaften von Benzimidazolyl-, Benzoxazolyl- und Benzothiazolyl-2,2'-bithienylverbindungen zu entnehmen.

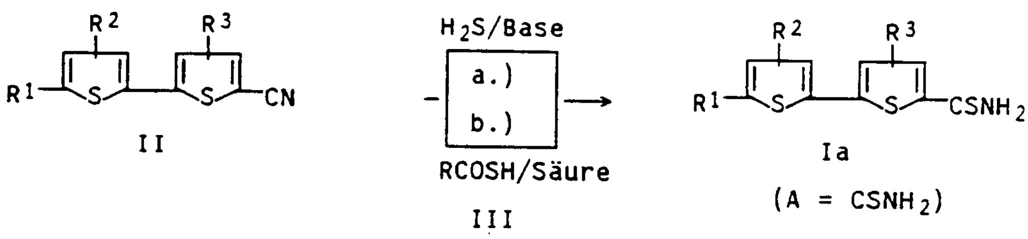


Die Umsetzung gemäß Reaktionsweg a.) wird im allgemeinen in einem inerten organischen Lösungsmittel wie Methanol, Ethanol, iso-Propanol, Diethylether, Tetrahydrofuran, Essigsäureethylester, Methylenchlorid, Hexan, Cyclohexan, Tetralin oder Toluol oder entsprechenden Gemischen bei Temperaturen von -15 °C bis 50 °C, vorzugsweise 10 °C bis 35 °C durchgeführt.

Als Basen eignen sich insbesondere tertiäre Amine wie Triethylamin, Dimethylphenylamin, N-Methylpiiperidin, N-Methylmorpholin und Pyridin. Besonders bevorzugt setzt man II und Schwefelwasserstoff in Gegenwart von Pyridin und/oder Triethylamin um.

Die Umsetzung von II mit einer Thiocarbonsäure III, in der R eine niedere Alkylgruppe wie Methyl, Ethyl und 1-Methylethyl bedeutet, gemäß Reaktionsweg b.) gelingt in Anlehnung an bekannte Methoden (The Chemistry of Amides; Interscience Publishers, New York 1970, Seite 417 ff) bevorzugt in einer niederen Carbonsäure wie Essigsäure und Propionsäure als Lösungsmittel in Gegenwart oder Abwesenheit von Wasser bei Temperaturen von 40 °C bis 160 °C, vorzugsweise 70 °C bis 130 °C.

Verbindungen Ia, in denen A Oxazol-2-yl oder Thiazol-2-yl bedeutet, erhält man beispielsweise, indem man ein entsprechendes Bithienylcarbonsäureamid IVa bzw. -thiocarbonsäureamid Ia in an sich bekannter Weise (Bull. Soc. Chim. France, 1974, S. 2079 ff) in einen inerten organischen Lösungsmittel mit einer α-Halogencarbonylverbindung V oder dem entsprechenden Acetal bzw. Ketal umsetzt.

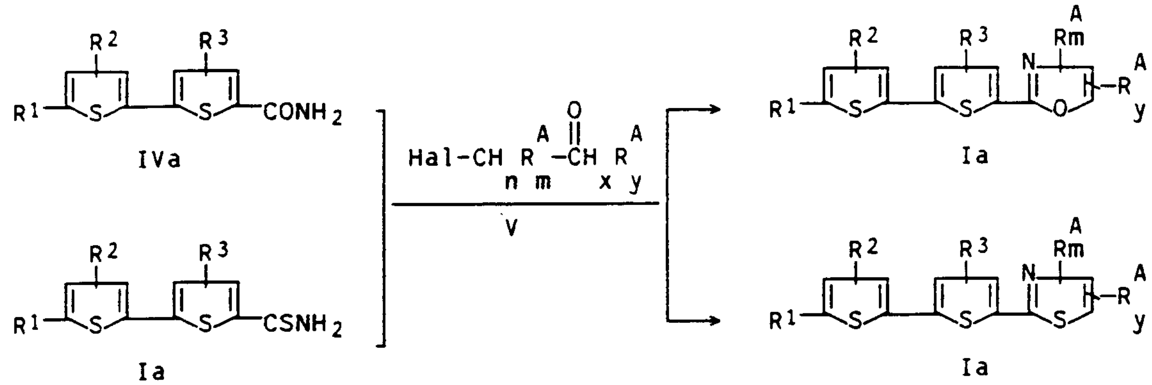


In Formel V bedeutet Hal ein Halogenatom wie Fluor, Chlor, Brom und Jod, vorzugsweise Chlor und Brom;

- $R^A$ einen beliebigen Substituenten des Restes A wie eingangs definiert, wobei mehrere dieser Substituenten in einem Molekül nicht die gleiche Bedeutung haben müssen,
- n 1 oder 2 mit $n + m = 2$ und
- x 0 oder 1 mit $x + y = 1$.

Als Lösungsmittel eignen sich hierbei insbesondere polare inerte organische Lösungsmittel wie Methanol, Ethanol, iso-Propanol, Dimethylformamid, Aceton, tert.-Butyl-methylketon und Acetonitril.

Die Umsetzungstemperatur liegt im allgemeinen bei 40 °C bis 170 °C, bevorzugt bei 60 °C bis 120 °C.

Außerdem kann es von Vorteil sein, zur Erhöhung der Raum/Zeit-Ausbeute der Reaktionsmischung eine Base wie die vorstehend genannten tertiären Amine und insbesondere Piperidin zuzusetzen und/oder die Umsetzung bei erhöhtem Druck (1 bis 30 atm, vorzugsweise 1 bis 5 atm) durchzuführen.

Verbindungen Ia, in denen A Oxazol-5-yl bedeutet erhält man, indem man ein entsprechendes Bithienylaldehydderivat VI in an sich bekannter Weise (J. Org. Chem., 42, 3114 f (1977)) in einem polaren organischen Lösungsmittel in Gegenwart einer Base mit einem entsprechenden Isonitril VIIa bzw. VIIb umsetzt.

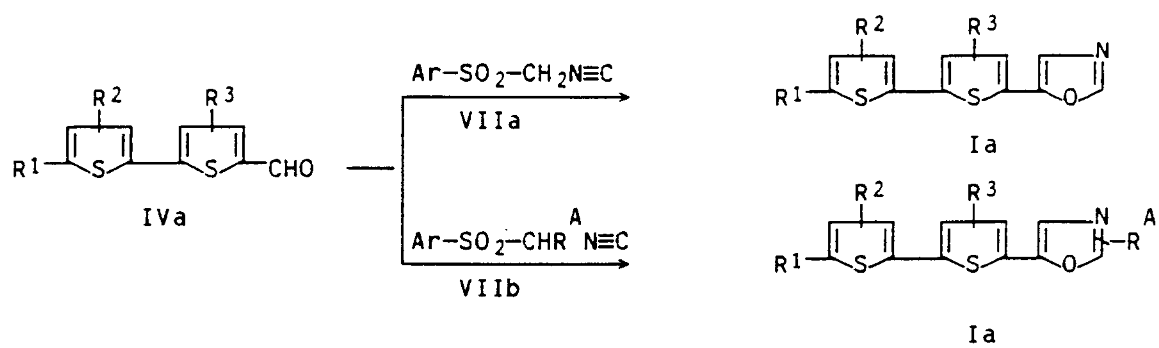


Ar in den Formeln VIIa und VIIb bedeutet eine Arylgruppe wie insbesondere Phenyl und Tolyl und $R^A$ hat die vorstehend bei der Verbindung V gegebene Bedeutung.

Insbesondere werden diese Umsetzungen in Methanol, Ethanol, iso-Propanol, Dimethylformamid, Aceton und/oder Acetonitril als Lösungsmittel in Anwesenheit einer organischen oder anorganischen Base wie die vorstehend genannten Amine und Methylate, Ethylate, tert.-Butylate, Hydroxide, Carbonate und Hydrogencarbonate von Alkalimetall- oder Erdalkalimetallkationen bei einer Temperatur von 30 °C bis 150 °C, vorzugsweise 50 °C bis 100 °C durchgeführt.

Verbindungen Ia, in denen A Isoxazol-3-yl bedeutet, erhält man in an sich bekannter Weise (Houben-Weyl, Bd. 10/3, S. 85 ff), indem man ein Bithienylnitriloxid VIII in einem inerten organischen Lösungsmittel mit Acetylen bzw. einem entsprechend substituierten Alkin IXa bzw. IXb umsetzt.

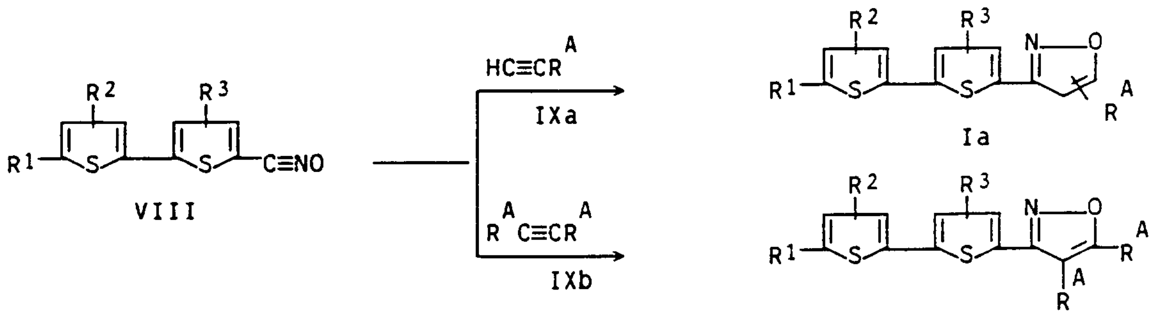

$R^A$ in den Formeln IXa und IXb hat die vorstehend bei Verbindung V gegebene Bedeutung. Im Rahmen dieser Defintion können die Reste $R^A$ in unterschiedlichen Positionen (Formel IXb und das entsprechende Umsetzungsprodukt) verschiedene Bedeutungen haben.

Für diese Umsetzung werden bevorzugt aprotische organische Lösungsmittel wie Methylenchlorid, Chloroform, Tetrachlorkohlenstoff, Chlorbenzol, Toluol, Xylol, Cyclohexan, Diethylether, Dioxan und Tetrahydrofuran eingesetzt.

Die Reaktionstemperatur liegt im allgemeinen bei -15 °C bis 70 °C, bevorzugt bei 10 °C bis 40 °C.

Die für die Umsetzung benötigten Bithienylnitriloxide VIII erhält man beispielsweise gemäß DE-A 27 54 832 aus den entsprechenden Oximen der Aldehyde VI. Wie in der zitierten Vorliteratur werden die Bithienylnitriloxide VIII ohne vorherige Isolierung, d.h. in situ mit den Acetylenen umgesetzt.

Verbindungen Ia, in denen A Pyrazol-5-yl bedeutet, erhält man in an sich bekannter Weise (The Chemistry of Heterocyclic Compounds - Pyrazoles etc, John Wiley and Sons, New York 1967, S. 10 ff) durch Umsetzung eines Bithienyldicarbonylderivates X mit Hydrazin oder einem Hydrazinderivat XI.

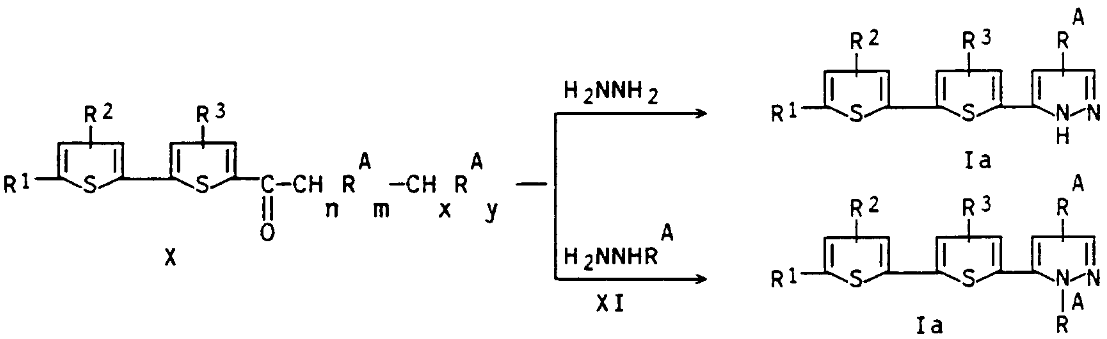

n, m, x, y und $R^A$ haben in den Formeln X und XI sowie deren Umsetzungsprodukt Ia die bei Formel V gegebene Bedeutung. Die Reste $R^A$ können im Rahmen ihrer Definition in unterschiedlichen Positionen eines Moleküls eine unterschiedliche Bedeutung haben.

Als Lösungsmittel für diese Umsetzung eignen sich besonders die vorstehend genannten protisch polaren Lösungsmittel, insbesondere Methanol, Ethanol, Essigsäure und/oder Propionsäure.

Die Umsetzungstemperatur liegt bei 50 °C bis 150 °C, vorzugsweise 80 °C bis 120 °C.

Die für die Umsetzung benötigten Bithienyldicarbonylverbindungen X erhält man nach bekannten Methoden wie durch Umsetzung einer entsprechenden Bithienylacylverbindung XIIa bzw. XIIb mit dem Acetal bzw. Ketal eines N,N-Dialkylcarbonsäureamids XIIIa bzw. XIIIb (US-A 3 086 854; Adv. org. Chem., 9, 393 ff (1979)).

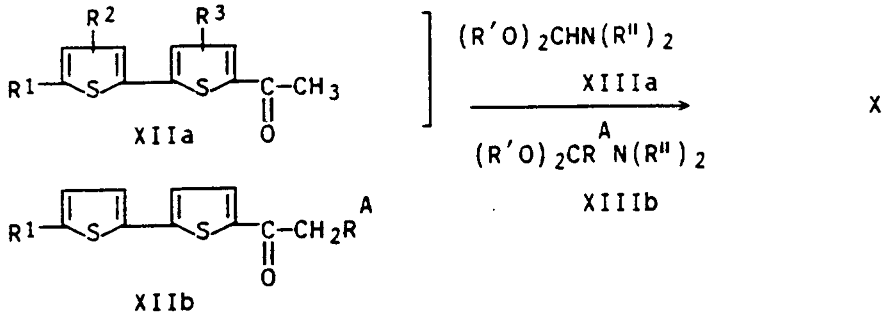


XIIa, XIIb, X

R' und R" in den Formeln XIIIa und XIIIb bedeuten $C_1$-$C_4$-Alkylgruppen wie insbesondere Methyl, Ethyl und iso-Propyl.

Zur Synthese der erfindungsgemäßen bzw. erfindungsgemäß verwendbaren Stoffe sind des weiteren Methoden nützlich, die in den nachstehenden Publikationen beschrieben sind:

Heterocycles, 10, 57 (1978);
Khim. Farm. ZH. 7(8), 13-17 (1973);
Khim. Farm. ZH. 6(6), 24-28 (1972);
Helv. Chim. Acta 1522 (1960);
Khim. Get. Soed. 770-772 (1972);
US 3,268,543; GB 1 268 817;
Synth Commun., 51 (1987);
Inorg. Chim, Acta 125, 203-206 (1986);
Bull. Chem. Soc. Jpn. 58, 2126 (1985);
J. Chem. Soc. Perkin II, 27 (1972);
Heterocycles 18, 117 (1982);
Tetrahedron 38(22), 3347 (1982);
J. Org. Chem. 47(8), 1590 (1982);
Bull. Soc. Chim. France, 2079 (1974).

Im Hinblick auf die biologische Wirksamkeit als Herbizide sind insbesondere Verbindungen I bevorzugt, in denen die Reste folgende Bedeutung haben:

$R^1$, $R^2$

Wasserstoff; Nitro; Formyl; Phenylcarbonyl;

Halogen wie Fluor, Chlor, Brom und Iod, vorzugsweise Chlor und Brom;

Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutxyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, vorzugsweise Trifluormethyl, vorzugsweise

Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl;

Cycloalkoxy wie Cyclopropyloxy, Cyclobutyloxy, Cyclopentyloxy, Cyclohexyloxy, Cycloheptyloxy und Cyclooctyloxy, vorzugsweise Cyclopropyloxy; Cyclopentyloxy und Cyclohexyloxy;

Alkoxy wie Methoxy, Ethoxy, Propoxy, 1-Methylethoxy, Butoxy, 1-Methylpropoxy, 2-Methylpropoxy und 1,1-Dimethylethoxy, vorzugsweise Methoxy, Ethoxy und Propoxy;

Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, vorzugsweise Trifluormethoxy;

Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethyltho;

Halogenalkylthio wie Difluormethylthio, Trifluormethylthio, Chlordifluormethylthio, Dichlorfluormethylthio, 2-Fluorethylthio, 2,2-Difluorethylthio, 1,1,2,2-Tetrafluorethylthio, 2,2,2-Trifluorethylthio und Pentafluorethylthio, vorzugsweise Difluormethylthio, Trifluormethylthio und Pentafluorethylthio;

Alkylcarbonyl wie Acetyl, Propionyl, iso-Propionyl, Butyryl, 2-Methylpropionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl,
3,3-Dimethylbutyryl und 2-Ethylbutyryl, vorzugsweise Acetyl, Propionyl, Butyryl und Pentanoyl, welches ein- bis dreifach durch die vorstehend genannten Halogenatome, insbesondere Fluor, Chlor und/oder Brom substituiert sein kann;
Cycloalkylcarbonyl wie Cyclopropylcarbonyl, Cyclobutylcarbonyl, Cyclopentylcarbonyl und Cyclohexylcarbonyl, vorzugsweise Cyclopentylcarbonyl und Cyclohexylcarbonyl, welches ein- bis dreifach durch Halogen wie vorstehend genannt insbesondere Fluor und/oder Chlor und/oder Alkyl wie vorstehend genannt mit ein bis vier Kohlenstoffatomen, insbesondere Methyl, Ethyl und/oder 1-Methylethyl substituiert sein kann;
Alkoxycarbonyl wie Methoxycarbonyl, Ethoxycarbonyl, Propyloxycarbonyl, 1-Methylethoxycarbonyl, Butyloxycarbonyl, 1-Methylpropylcarbonyl, 2-Methylpropylcarbonyl und 1,1-Dimethylethoxycarbonyl, vorzugsweise Methoxy- und Ethoxycarbonyl;
$R^3$
Wasserstoff, Halogen, Alkyl, Alkoxy, Alkylthio, Halogenalkyl oder Halogenalkoxy mit jeweils ein bis vier Kohlenstoffatomen, wie unter $R^1$ definiert, vorzugsweise Wasserstoff, Fluor, Chlor, Brom, Methyl, Ethyl, Propyl, Butyl, Methoxy, Ethoxy, Methylthio, Difluormethyl, Trifluormethyl, Difluormethoxy oder Trifluormethoxy;
A
$-CSNH_2$;
oder ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome, wie bei $R^1$ genannt, insbesondere Fluor und/oder Chlor und/oder ein bis zwei der folgenden Reste tragen können:
Alkyl, Alkoxy und/oder Alkylthio, insbesondere wie bei $R^3$ genannt, welche ihrerseits ein- bis fünffach durch insbesondere Fluor und/oder Chlor und/oder einfach durch Alkoxy wie insbesondere Methoxy oder Ethoxy, Halogenalkoxy wie insbesondere Difluormethoxy oder Trifluormethoxy, Alkylthio wie insbesondere Methylthio oder Ethylthio, Amino, Alkylamino wie insbesondere Methylamino und Ethylamino oder Dialkylamino wie Dimethylamino oder Diethylamino substituiert sein können,

Cycloalkyl mit drei bis sechs Kohlenstoffatomen wie inbesondere bei $R^3$ genannt und/oder Phenyl, welche ihrerseits ein bis fünf Halogenatome wie insbesondere Fluor und Chlor und/oder ein bis drei der bei $R^3$ insbesondere genannten Reste,

ein Rest $-NR^4R^5$ oder ein Rest $-COR^6$; $R^4$, $R^5$
Wasserstoff; Formyl;
Alkyl wie bei $R^1$ genannt, insbesondere Methyl, Ethyl und/oder 1-Methylethyl;
Halogenalkyl wie im allgemeinen und im besonderen bei $R^1$ genannt;
Alkoxy wie im allgemeinen und im besonderen bei $R^3$ genannt;
Halogenalkoxy wie bei $R^3$ genannt, insbesondere Difluormethoxy und Trifluormethoxy;
Alkylcarboxyl wie im allgemeinen und im besonderen bei $R^1$ genannt, welches im Alkylteil ein bis drei Halogenatome wie insbesondere Fluor und/oder Chlor tragen kann;
Alkoxycarbonyl wie bei $R^1$ genannt, insbesondere Methoxycarbonyl, Ethoxycarbonyl, 1-Methylethoxycarbonyl und 1,1-Dimethylethoxycarbonyl und/oder Phenylcarbonyl, welches seinerseits ein bis fünf Halogenatome wie insbesondere Fluor und Chlor und/oder ein bis drei der folgenden Gruppen tragen kann: Alkyl wie insbesondere Methyl und Ethyl, Halogenalkyl wie insbesondere Difluormethyl und Trifluormethyl, Alkoxy wie insbesondere Methoxy und 1-Methylethoxy und/oder Halogenalkoxy wie insbesondere Difluormethoxy und Trifluormethoxy;
$R^6$
Hydroxy;
Alkoxy wie bei $R^1$ genannt, insbesondere Methoxy, Ethoxy und 1-Methylethoxy;
Alkoxyalkoxy wie 2-(Methoxy)ethoxy, 2-(Ethoxy)ethoxy, 2-(Methoxy)-propyloxy, 3-(Methoxy)propyloxy, 2-(Ethoxy)-propyloxy, 3-(Ethoxy)-propyloxy, 1-Methyl-2-methoxyethoxy, 2-Ethoxy-1-methylethoxy, 2-Methoxy-1-methylpropyloxy, 2-Ethoxy-1-methylpropyloxy und 2-Methoxy-2-methylpropyloxy, insbesondere 2-Methoxyethoxy, 2-Ethoxyethoxy, 2-Methoxypropyloxy und 2-Methoxy-1-methylethoxy;
Alkenyloxy wie Allyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1,2-Dimethyl-2-propenyloxy, 1,1-Dimethyl-2-propenyloxy, 1-Methyl-3-butenyloxy, 1-Ethyl-2-propenyloxy, 2-Hexenyloxy, 1-Methyl-2-pentenyloxy, 1-Ethyl-2-butenyl und 2-Ethyl-2-butenyloxy,vorzugsweise Allyloxy, 2-Butenyl und 3-Butenyl;
Alkinyloxy wie 2-Propinyloxy, 2-Butinyloxy, 3-Butinyloxy, 1-Methyl-2-propinyloxy, 1-Methyl-2-butinyloxy, 1-Ethyl-2-propinyloxy, 2-Pentinyloxy, 3-Pentinyloxy, 4-Pentinyl und 1-Methyl-2-pentinyloxy, bevorzugt 2-Propinyloxy;

Phenoxy oder Benzyloxy, wobei die aromatischen Ringe ihrerseits ein- bis fünffach durch Halogen wie vorstehend bei $R^1$ genannt, insbesondere Fluor und Chlor und/oder ein- bis dreifach durch Alkyl, Halogenalkyl, Alkoxy oder Halogenalkoxy wie bei $R^1$ genannt, insbesondere mit ein oder zwei Kohlenstoffatomen substituiert sein können;

oder eine Gruppe $NR^7,R^8$;

$R^7$, $R^8$

Wasserstoff;

Alkyl wie bei $R^1$ im allgemeinen und im besonderen genannt;

Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl und 3-Butenyl;

Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl;

Alkoxyalkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl;

Phenyl oder Benzyl.

Die Bithienylderivate Ia bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen Ia können beispielsweise wie folgt formuliert werden:

I. Man vermischt 90 Gewichtsteile der Verbindung Nr. 1.002 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2.001 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 6.006 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 7.001 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |

| Botanischer Name | Deutscher Name |
| --- | --- |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |

| Botanischer Name | Deutscher Name |
|---|---|
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Bithienylderivate Ia mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die herbiziden Verbindungen Ia allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen Ia benutzt. Die so oder gemäß der zitierten Literatur (WO-A 86/05949) erhaltenen Verbindungen sind in den nachstehenden Tabellen mit physikalischen Angaben aufgeführt. Verbindungen ohne diese Angaben lassen sich auf analogem Weg erhalten. Sie lassen aufgrund ihrer engen strukturellen Beziehung eine ähnliche Wirkung erwarten.

Beispiel 1

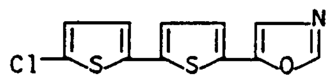

20 mmol 5'-Chlor-bithienyl-5-carbaldehyd und 20 mmol p-Toluolsulfonylmethylisocyanit (TosMIC) wurden in 60 ml Methanol dispergiert und der so erhaltenen Mischung wurden bei 20 °C 40 mmol Kaliumcarbonat zugegeben. Das Reaktionsgemisch wurde 30 Minuten unter Rückfluß erhitzt. Durch Zugabe von Wasser zur erkalteten Reaktionsmischung fiel das gewünschte Produkt als Feststoff an.
(Ausbeute 60 %; Fp. 89 °C; Wirkstoff Nr. 1.002).

Beispiel 2

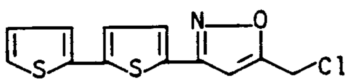

Eine Mischung aus 8,1 g Bithienyl-5-carbaldoxim und 50 ml Methylenchlorid wurde nacheinander bei 0 °C zuerst mit 5,0 g Propargylchlorid und danach mit 31,5 g 10 %iger Natriumhypochlorit-Lösung (in Wasser) und 0,5 g Natriumhydroxid versetzt und die so erhaltene Reaktionsmischung 10 Stunden bei 20 °C belassen. Durch Aufarbeitung der organischen Phase in üblicher Weise und anschließende Chromatographie wurde das Produkt isoliert.
(Ausbeute 6,9 g; Fp. 80 °C; Wirkstoff Nr. 2.002).

Beispiel 3

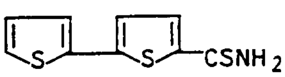

Eine Lösung aus 47,5 g Bithienyl-5-carbaldehyd, 400 ml Ether und 30 ml Methylenchlorid wurde bei -5 °C nacheinander zuerst mit 300 ml Eis und dann mit 35 g Hydroxylamin-O-sulfat versetzt. Nach 1 Stunde rühren bei -5 °C wurde das organische Lösungsmittel unter Kühlung bei vermindertem Druck entfernt. Die wäßrige Phase wurde mit Hilfe von Aktivkohle gereinigt und anschließend bei 0 °C in 250 ml 4n Natronlauge eingerührt, wobei Bithienyl-5-cyanid ausfiel.

20 g des so erhaltenen Cyanids, 4 ml Eisessig und 44 g Thioessigsäure wurden 0,5 Stunden bei 75 °C gerührt. Das Produkt wurde durch Ausrühren mit 250 ml Iso-Propanol/Wasser (1:1) aus der Reaktionsmischung isoliert.
(Ausbeute 15,1 g; Fp. >200 °C; Wirkstoff Nr. 7.001).

Beispiel 4

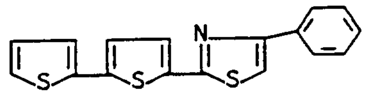

Eine Mischung aus 3,0 g α-Bromacetophenon, 3,0 g Bithienyl-5-thioamid (Beispiel 4), 2 Tropfen Piperidin und 50 ml Iso-Propanol wurde 0,5 h unter Rückfluß erhitzt. Das Produkt fiel beim Abkühlen der Reaktionsmischung als Feststoff aus.
(Ausbeute 2,95 g; Fp. 139-141 °C; Wirkstoff Nr. 6.007).

Tabelle 1:

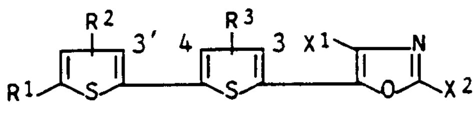

I.1

| Nr. | $R^1$ | $R^2$ | $R^3$ | $X^1$ | $X^2$ | phys. Daten |
|---|---|---|---|---|---|---|
| 1.002 | H | H | H | H | H | Fp. 80-81°C |
| 1.002 | Cl | H | H | H | H | Fp. 89°C |
| 1.003 | H | H | H | H | $CH_3$ | |
| 1.004 | H | H | H | H | $CH_2CH_3$ | |
| 1.005 | Cl | H | H | H | $CH_3$ | |
| 1.006 | H | H | H | $CH_3$ | H | |
| 1.007 | Cl | H | H | H | H | |
| 1.008 | $CH_2CH_3$ | H | H | H | H | |
| 1.009 | Cl | H | H | H | $CH_3$ | |
| 1.010 | Br | H | H | H | H | |
| 1.011 | H | H | H | H | $C_6H_5$ | |
| 1.012 | Cl | H | H | H | H | |
| 1.013 | $CH_3$ | 4'-$CH_3$ | H | H | H | |
| 1.014 | $CH_3$ | 4'-$CH_3$ | H | H | $CH_3$ | |
| 1.015 | H | H | 4-$CH_3$ | H | H | |
| 1.016 | $COOCH_3$ | H | H | H | H | |
| 1.017 | COOH | H | H | H | H | |
| 1.018 | $COOCH_2CH_3$ | H | H | H | H | |
| 1.019 | H | H | H | H | $CF_3$ | |
| 1.020 | H | H | H | $CH_2CH_3$ | H | |
| 1.021 | H | H | H | H | 4-Cl-$C_6H_4$ | |

Tabelle 2:

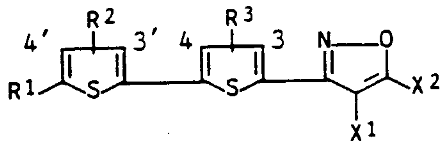

I.2

| Nr. | $R^1$ | $R^2$ | $R^3$ | $X^1$ | $X^2$ | phys. Daten |
|---|---|---|---|---|---|---|
| 2.001 | H | H | H | H | H | Fp. 93-95°C |
| 2.002 | H | H | H | H | $CH_2Cl$ | Fp. 80°C |
| 2.003 | Cl | H | H | H | H | |
| 2.004 | Br | H | H | H | H | |
| 2.005 | Cl | H | H | H | $CH_2Cl$ | |
| 2.006 | Br | H | H | H | $CH_2Cl$ | |
| 2.007 | H | H | H | H | $COOCH_2CH_3$ | |
| 2.008 | H | H | H | H | $COOCH_3$ | |
| 2.009 | H | H | H | H | $C_6H_5$ | |
| 2.010 | H | H | H | $CH_3$ | H | |
| 2.011 | $CH_3$ | H | H | H | H | |
| 2.012 | $CH_2CH_3$ | H | H | H | H | |
| 2.013 | $CH_3$ | H | H | H | $CH_2Cl$ | |
| 2.014 | H | H | 4-$CH_3$ | H | H | |
| 2.015 | H | H | 4-$CH_2CH_3$ | H | H | |
| 2.016 | $CH_3$ | H | 4-$CH_3$ | H | H | |
| 2.017 | COOH | H | H | H | H | |
| 2.018 | $COOCH_3$ | H | H | H | H | |
| 2.019 | $COOCH_2CH_3$ | H | H | H | H | |
| 2.020 | $CONH_2$ | H | H | H | H | |
| 2.021 | H | H | H | H | $CH_2OH$ | |

Tabelle 3:

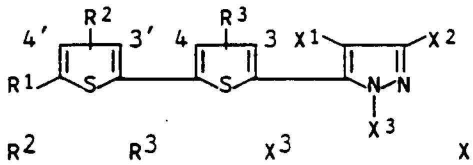

I.3

| Nr. | $R^1$ | $R^2$ | $R^3$ | $X^3$ | $X^1$ | $X^2$ | phys. Daten |
|---|---|---|---|---|---|---|---|
| 3.001 | H | H | H | $C_6H_5$ | H | H | |
| 3.002 | H | H | H | $4-Cl-C_6H_4$ | H | H | |
| 3.003 | H | H | H | $4-F-C_6H_4$ | H | H | |
| 3.004 | H | H | H | $4-CH_3-C_6H_4$ | H | H | |
| 3.005 | H | H | H | 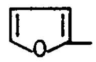 | H | H | |
| 3.006 | H | H | H | 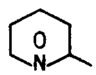 | H | H | |
| 3.007 | H | H | H | H | $CH_3$ | H | |
| 3.008 | H | H | H | H | H | $CH_3$ | |
| 3.009 | H | H | H | H | H | $C_6H_5$ | |
| 3.010 | Cl | H | H | H | H | | |
| 3.011 | $CH_3$ | H | H | H | H | H | |
| 3.012 | $CH_2CH_3$ | H | H | H | H | H | |
| 3.013 | Br | H | H | H | H | H | |
| 3.014 | Cl | H | H | H | $CH_3$ | $CH_3$ | |
| 3.015 | Cl | H | H | $C_6H_5$ | $CH_3$ | $CH_3$ | |
| 3.016 | H | H | $4-CH_3$ | $CH_3$ | H | H | |
| 3.017 | Cl | H | H | $CH_3$ | H | H | |
| 3.018 | $CH_3$ | H | H | $CH_3$ | H | H | |
| 3.019 | $CH_3$ | H | H | $C_6H_5$ | $CH_3$ | $CH_3$ | |
| 3.020 | $OCH_3$ | H | H | $CH_3$ | H | H | |

Tabelle 4:

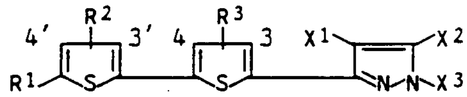

| Nr. | $R^1$ | $R^2$ | $R^3$ | $X^3$ | $X^1$ | $X^2$ | phys. Daten |
|---|---|---|---|---|---|---|---|
| 4.001 | H | H | H | $C_6H_5$ | H | H | |
| 4.002 | H | H | H | $4-Cl-C_6H_4$ | H | H | |
| 4.003 | H | H | H | $4-F-C_6H_4$ | H | H | |
| 4.004 | H | H | H | $4-CH_3-C_6H_4$ | H | H | |
| 4.005 | H | H | H | 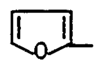 | H | H | |
| 4.006 | H | H | H | 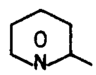 | H | H | |
| 4.007 | H | H | H | H | $CH_3$ | H | |
| 4.008 | H | H | H | H | H | $CH_3$ | |
| 4.009 | H | H | H | H | $CH_3$ | $CH_3$ | |
| 4.010 | Cl | H | H | H | H | H | |
| 4.011 | $CH_3$ | H | H | H | H | H | |
| 4.012 | $CH_2CH_3$ | H | H | H | H | H | |
| 4.013 | Br | H | H | H | H | H | |
| 4.014 | Cl | H | H | H | $CH_3$ | $CH_3$ | |
| 4.015 | Cl | H | H | $C_6H_5$ | $CH_3$ | $CH_3$ | |
| 4.016 | H | 4'-$CH_3$ | H | $CH_3$ | H | $CH_3$ | |
| 4.017 | Cl | H | H | $CH_3$ | H | H | |
| 4.018 | Cl | H | H | $CH_3$ | H | $CH_3$ | |
| 4.019 | $CH_3$ | H | H | $C_6H_5$ | $CH_3$ | $CH_3$ | |
| 4.020 | $OCH_3$ | H | H | H | H | H | |
| 4.021 | $CH_3$ | H | 4-$CH_3$ | H | H | H | |

Tabelle 5:

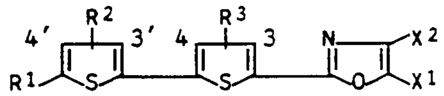

I.5

| Nr. | $R^1$ | $R^2$ | $R^3$ | $X^1$ | $X^2$ | phys. Daten |
|---|---|---|---|---|---|---|
| 5.001 | H | H | H | H | H | |
| 5.002 | H | H | H | $CH_3$ | H | |
| 5.003 | H | H | H | H | $CH_3$ | |
| 5.004 | $CH_3$ | H | H | H | $CH_3$ | |
| 5.005 | H | H | 4-$CH_3$ | H | H | |
| 5.006 | H | 4'-$CH_3$ | H | H | H | |
| 5.007 | Cl | H | H | H | $CH_3$ | |
| 5.008 | $CH_2CH_3$ | H | H | H | $CH_3$ | |
| 5.009 | Br | H | H | H | $CH_3$ | |
| 5.010 | H | H | H | $C_6H_5$ | H | |
| 5.011 | Cl | H | 4-Cl | H | $CH_3$ | |
| 5.012 | $OCH_3$ | H | H | H | $CH_3$ | |
| 5.013 | H | H | H | $CH_3$ | $CH_3$ | |
| 5.014 | Cl | H | H | $CH_3$ | $CH_3$ | |
| 5.015 | Cl | 4'-$CH_3$ | H | $CH_3$ | $CH_3$ | |
| 5.016 | H | H | H | $CH_2CH_3$ | $CH_2CH_3$ | |
| 5.017 | H | H | H | H | $CH_2C_6H_5$ | |
| 5.018 | H | H | H | $CH_2CH_3$ | H | |
| 5.019 | H | H | H | $CH_2OCH_3$ | H | |
| 5.020 | Cl | H | H | $CH_2OCH_3$ | H | |

Tabelle 6:

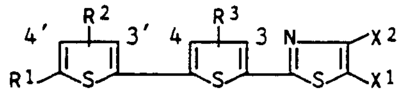

I.6

| Nr. | $R^1$ | $R^2$ | $R^3$ | $X^1$ | $X^2$ | phys. Daten |
|---|---|---|---|---|---|---|
| 6.001 | H | H | H | H | H | |
| 6.002 | H | H | H | H | $CH_3$ | |
| 6.003 | Cl | H | H | H | $C(CH_3)_3$ | |
| 6.004 | Cl | H | H | H | $C_6H_5$ | |
| 6.005 | H | H | H | H | $COOCH_2CH_3$ | Fp. 86-88 °C |
| 6.006 | H | H | H | H | $4-F-C_6H_4$ | Fp. 155-157°C |
| 6.007 | H | H | H | H | $C_6H_5$ | Fp. 139-141°C |
| 6.008 | H | H | H | H | $C(CH_3)_3$ | Fp. 81-83°C |
| 6.009 | H | H | H | $CH_3$ | $C_6H_5$ | Fp. 106-108°C |
| 6.010 | Br | H | H | H | H | |
| 6.011 | $CH_3$ | H | H | H | H | |
| 6.012 | $CH_3$ | H | H | H | $C_6H_5$ | |
| 6.013 | H | 4'-$CH_3$ | H | H | $C_6H_5$ | |
| 6.014 | Cl | H | 4-Cl | H | H | |
| 6.015 | Cl | H | 4-Cl | H | $C_6H_5$ | |
| 6.016 | $CH_3$ | H | 4-$CH_3$ | H | H | |
| 6.017 | $CH_3$ | H | 4-$CH_3$ | H | $C_6H_5$ | |
| 6.018 | H | H | H | H | $CH_3$ | |
| 6.019 | H | H | H | H | $CH_2Cl$ | |
| 6.020 | Cl | H | H | H | $CH_2Cl$ | |
| 6.021 | $CH_3$ | H | H | H | $CH_2Cl$ | |
| 6.022 | $OCH_3$ | H | H | H | $C_6H_5$ | |

Tabelle 7:

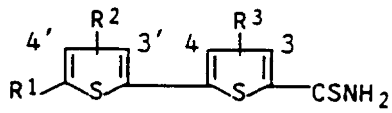

I.7

| Nr. | $R^1$ | $R^2$ | $R^3$ | phys. Daten |
|---|---|---|---|---|
| 7.001 | H | H | H | Fp. > 200°C |
| 7.002 | $CH_3$ | H | H | |
| 7.003 | $CH_2CH_3$ | H | H | |
| 7.004 | 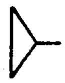 | H | H | |
| 7.005 | $OCH_3$ | H | H | |
| 7.006 | $OCH_2CH_3$ | H | H | |
| 7.007 | Cl | H | 4-Cl | |
| 7.008 | Cl | H | H | Fp. > 200 °C |
| 7.009 | H | 4'-$CH_3$ | H | |
| 7.010 | Cl | 4'-$CH_3$ | H | |
| 7.011 | H | H | 4-$CH_3$ | |
| 7.012 | Br | H | H | |
| 7.013 | $COOCH_3$ | H | H | |
| 7.014 | Cl | 4'-Cl | 4-Cl | |
| 7.015 | $COOCH_3$ | H | H | |
| 7.016 | $COOCH_2CH_3$ | H | H | |

Anwendungsbeispiele

Die Wirkung der herbiziden Bithienylderivate der Formel Ia auf das Pflanzenwachstum läßt sich durch Gewächshausversuche zeigen:

Zur Anzucht der Testpflanzen dienen Plastikblumentöpfe mit 300 $cm^3$ Inhalt und lehmigem Sand mit etwa 3 % Humus als Substrat. Die Samen der Testpflanzen werden nach Arten getrennt flach eingesät.

Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewaschsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung mit den in Wasser suspendierten oder emulgierten Wirkstoffen in die Versuchsgefäße verpflanzt. Die Aufwandmenge für die Nachauflaufbehandlung beträgt 0,5 kg/ha und 1,0 kg/ha aktive Substanz (a.S.). Eine Abdeckung unterbleibt

bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Bromus inermis | unbegrannte Trespe |
| Digitaria sanguinalis | Blutfingerhirse |
| Linum usitatissimum | Lein |
| Oryza sativa | Reis |
| Solanum nigrum | Schwarzer Nachtschatten |
| Stellaria media | Vogelsternmiere |
| Veronica spp. | Ehrenpreisarten |

Mit 0,5 kg/ha a.S. im Nachauflaufverfahren eingesetzt, lassen sich mit Beispiel 1.001 bzw. Beispiel 1.002 unerwünschte Pflanzen sehr gut bekämpfen, und zwar mit gleichzeitiger Verträglichkeit für Reis.

Dergleichen hat Verbindung Nr. 2.002 mit 1,0 kg/ha a.S. bei Nachauflaufanwendung herbizide Wirkung gegen Amaranthus retroflexus und Digitaria sanguinalis.

**Patentansprüche**

**Patentansprüche für folgende Vertragsstaaten : CH, DE, FR, GB, IT, LI, NL**

**1.** Bithienylderivate der allgemeinen Formel Ia,

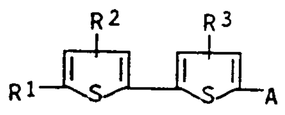

Ia

in der die Substituenten folgende Bedeutung haben:

$R^1$, $R^2$ Wasserstoff, Halogen, Nitro, Formyl, $C_1$-$C_6$-Alkyl, $C_1$-$C_6$-Halogenalkyl, $C_3$-$C_8$-Cycloalkyl, $C_3$-$C_8$-Cycloalkoxy, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_6$-Alkylcarbonyl, welches ein- bis dreifach durch Halogenatome substituiert sein kann, $C_3$-$C_6$-Cycloalkylcarbonyl, welches ein- bis dreifach durch Halogen und/oder $C_1$-$C_4$-Alkyl substituiert sein kann, $C_1$-$C_6$-Alkoxycarbonyl und/oder Phenylcarbonyl;

$R^3$ Wasserstoff, ein Halogenatom, eine $C_1$-$C_4$-Alkylgruppe, eine $C_1$-$C_4$-Alkoxygruppe, eine $C_1$-$C_4$-Alkylthiogruppe, eine $C_1$-$C_4$-Halogenalkylgruppe oder eine $C_1$-$C_4$-Halogenalkoxygruppe;

A ein Rest $-CSNH_2$;
oder ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Substituenten tragen können:

- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder

- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

**2.** Verfahren zur Herstellung der Verbindungen Ia gemäß Anspruch 1, in denen A eine Gruppe $-CSNH_2$ bedeutet, dadurch gekennzeichnet, daß man ein Bithienylcyanid II

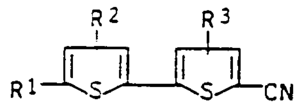

in an sich bekannter Weise
a) in Gegenwart einer Base mit Schwefelwasserstoff oder
b) in Gegenwart einer Säure mit einer Thiocarbonsäure III

RCOSH III,

in der R für eine niedere Alkylgruppe wie Methyl, Ethyl oder 1-Methylethyl steht,
umsetzt.

**3.** Verfahren zur Herstellung der Verbindungen Ia gemäß Anspruch 1, in denen A Oxazol-2-yl oder Thiazol-2-yl bedeutet, dadurch gekennzeichnet, daß man ein Bithienylcarbonsäureamid IVa bzw. ein Bitheinylthiocarbonsäureamid Ia ($A = CSNH_2$)

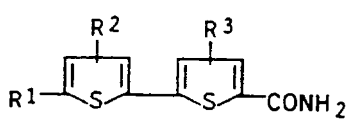

IVa

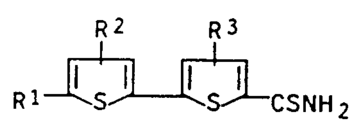

Ia

in an sich bekannter Weise in einem inerten organischen Lösungsmittel mit einer α-Halogencarbonylverbindung der Formel V

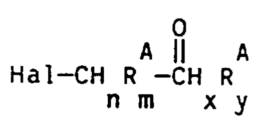

V

in der Hal ein Halogenatom und $R^A$ einen beliebigen Substituenten des Restes A entsprechend Anspruch 1 bedeutet, n 1 oder 2 bedeutet und n + m den Wert 2 hat, x 0 oder 1 bedeutet und x + y den Wert 1 hat, oder einem entsprechenden Acetal bzw. Ketal umsetzt.

4. Verfahren zur Herstellung der Verbindungen Ia gemäß Anspruch 1, in denen A Oxazol-5-yl bedeutet, dadurch gekennzeichnet, daß man ein Bithienylaldehydderivat VI

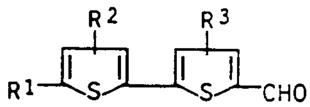

VI

in an sich bekannter Weise in einem inerten organischen Lösungsmittel in Gegenwart einer Base mit einem entsprechenden Isonitril der Formel VIIa bzw. VIIb

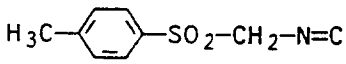

VIIa

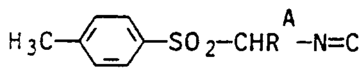

VIIb

in der $R^A$ einen beliebigen Substituenten des Restes A entsprechend Anspruch 1 bedeutet,umsetzt.

5. Verfahren zur Herstellung der Verbindungen Ia gemäß Anspruch 1, in denen A Isoxazol-3-yl bedeutet, dadurch gekennzeichnet, daß man ein Bithienylnitriloxid VIII

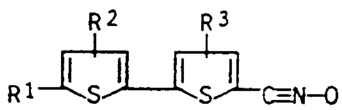

VIII

in an sich bekannter Weise in einem inerten organischen Lösungsmittel mit Acetylen oder einem entsprechenden Alkin der Formel IXa bzw. IXb,

$HC\equiv CR^A$

IXa

$R^A C\equiv CR^A$

IXb

in der $R^A$ die in Anspruch 3 gegebene Bedeutung hat, umsetzt.

6. Verfahren zur Herstellung der Verbindungen Ia gemäß Anspruch 1, in denen A Pyrazol-5-yl bedeutet, dadurch gekennzeichnet, daß man ein Bithienyldicarbonylderivat X,

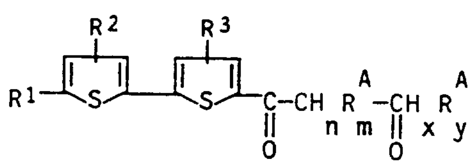

X

in der n, m, x, y und $R^A$ die in Anspruch 3 gegebene Bedeutung haben, in an sich bekannter Weise in einem inerten organischen Lösungsmittel mit Hydrazin oder einem Hydrazinderivat der Formel XI

$H_2N\text{-}NHR^A$ XI

umsetzt.

7. Herbizide Mittel, enthaltend ein Bithienylderivat der allgemeinen Formel Ia gemäß Anspruch 1 und übliche inerte Zusatzstoffe.

8. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Bithienylderivats der allgemeinen Formel Ia gemäß Anspruch 1 behandelt.

9. Verwendung von Bithienylderivaten der Formel Ia gemäß Anspruch 1 als herbizide Mittel.

**Patentansprüche für folgenden Vertragsstaat : ES**

1. Verfahren zur Herstellung der Bithienylderivate der allgemeinen Formel Ia,

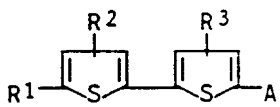

Ia

in der die Substituenten folgende Bedeutung haben:

$R^1$, $R^2$ Wasserstoff, Halogen, Nitro, Formyl, $C_1$-$C_6$-Alkyl, $C_1$-$C_6$-Halogenalkyl, $C_3$-$C_8$-Cycloalkyl, $C_3$-$C_8$-Cycloalkoxy, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_6$-Alkylcarbonyl, welches ein- bis dreifach durch Halogenatome substituiert sein kann, $C_3$-$C_6$-Cycloalkylcarbonyl, welches ein- bis dreifach durch Halogen und/oder $C_1$-$C_4$-Alkyl substituiert sein kann, $C_1$-$C_6$-Alkoxycarbonyl und/oder Phenylcarbonyl;

$R^3$ Wasserstoff, ein Halogenatom, eine $C_1$-$C_4$-Alkylgruppe, eine $C_1$-$C_4$-Alkoxygruppe, eine $C_1$-$C_4$-Alkylthiogruppe, eine $C_1$-$C_4$-Halogenalkylgruppe oder eine $C_1$-$C_4$-Halogenalkoxygruppe;

A ein Rest $-CSNH_2$;

dadurch gekennzeichnet, daß man ein Bithienylcyanid II

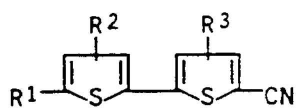

in an sich bekannter Weise

a) in Gegenwart einer Base mit Schwefelwasserstoff oder

b) in Gegenwart einer Säure mit einer Thiocarbonsäure III

RCOSH

in der R für eine niedere Alkylgruppe wie Methyl, Ethyl oder 1-Methylethyl steht,

umsetzt.

2. Verfahren zur Herstellung der Verbindungen Ia gemäß Anspruch 1, in denen A ein heterocyclischer Rest, ausgewählt aus der Gruppe Thiazol-2-yl, Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Sustituenten tragen können:

- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder

- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl

bedeutet, dadurch gekennzeichnet, daß man ein Bithienylcarbonsäureamid IVa bzw. ein Bithienylthiocarbonsäureamid Ia ($A = CSNH_2$)

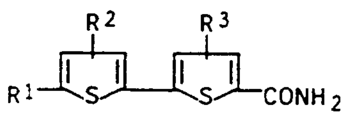

IVa

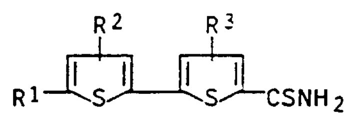

Ia

in an sich bekannter Weise in einem inerten organischen Lösungsmittel mit einer α-Halogencarbonylverbindung der Formel V

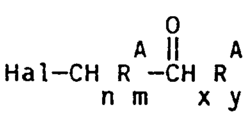

V

in der Hal ein Halogenatom und $R^A$ einen beliebigen Substituenten des Restes A entsprechend Anspruch 1 bedeutet, n 1 oder 2 bedeutet und n + m den Wert 2 hat, x 0 oder 1 bedeutet und x + y den Wert 1 hat, oder einem entsprechenden Acetal bzw. Ketal umsetzt.

**3.** Verfahren zur Herstellung der Verbindungen Ia gemäß Anspruch 1, in denen A ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Sustituenten tragen können:

- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder
- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

bedeutet, dadurch gekennzeichnet, daß man ein Bithienylaldehydderivat VI

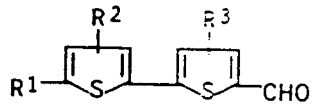

VI

in an sich bekannter Weise in einem inerten organischen Lösungsmittel in Gegenwart einer Base mit einen entsprechenden Isonitril der Formel VIIa bzw. VIIb

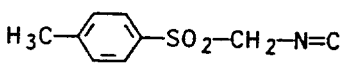

VIIa

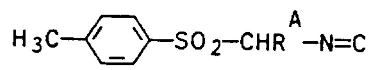

**VIIb**

in der $R^A$ einen beliebigen Substituenten des Restes A entsprechend Anspruch 1 bedeutet, umsetzt.

**4.** Verfahren zur Herstellung der Verbindungen Ia gemäß Anspruch 1, in denen A ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Substituenten tragen können:

- $C_1$-$C_6$-Alkyl, $C_1$-$C_6$-Alkoxy, $C_1$-$C_5$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder
- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

bedeutet, dadurch gekennzeichnet, daß man ein Bithienylnitriloxid VIII

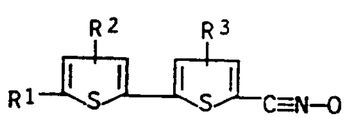

VIII

in an sich bekannter Weise in einem inerten organischen Lösungsmittel mit Acetylen oder einem entsprechenden Alkin der Formel IXa bzw. IXb,

$HC\equiv CR^A$

IXa

$R^A C\equiv CR^A$

IXb

in der $R^A$ die in Anspruch 2 gegebene Bedeutung hat, umsetzt.

**5.** Verfahren zur Herstellung der Verbindungen Ia gemäß Anspruch 1, in denen A ein hetrocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Substituenten tragen können:

- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest -$NR^4R^5$ oder
- ein Rest -$COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe -$NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

bedeutet, dadurch gekennzeichnet, daß man ein Bithienyldicarbonylderivat X,

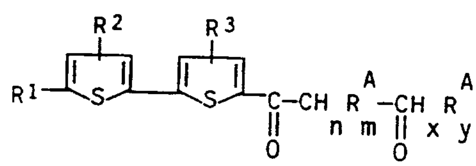

X

in der n, m, x, y und $R^A$ die in Anspruch 2 gegebene Bedeutung haben, in an sich bekannter Weise in einem inerten organischen Lösungsmittel mit Hydrazin oder einem Hydrazinderivat der Formel XI

$H_2N$-$NHR^A$ XI

umsetzt.

**6.** Herbizide Mittel, enthaltend übliche inerte Zusatzstoffe und ein Bithienylderivat der allgemeinen Formel Ia

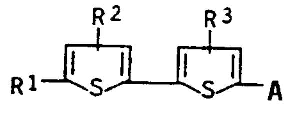

Ia

in der die Substituenten folgende Bedeutung haben:

$R^1$, $R^2$ Wasserstoff, Halogen, Nitro, Formyl, $C_1$-$C_6$-Alkyl, $C_1$-$C_6$-Halogenalkyl, $C_3$-$C_8$-Cycloalkyl, $C_3$-$C_8$-Cycloalkoxy, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_6$-Alkylcarbonyl, welches ein- bis dreifach durch Halogenatome substituiert sein kann, $C_3$-$C_6$-Cycloalkylcarbonyl, welches ein- bis dreifach durch Halogen und/oder $C_1$-$C_4$-Alkyl substituiert sein kann, $C_1$-$C_6$-Alkoxycarbonyl und/oder Phenylcarbonyl;

$R^3$ Wasserstoff, ein Halogenatom, eine $C_1$-$C_4$-Alkylgruppe, eine $C_1$-$C_4$-Alkoxygruppe eine $C_1$-$C_4$-Alkylthiogruppe, eine $C_1$-$C_4$-Halogenalkylgruppe oder eine $C_1$-$C_4$-Halogenalkoxy-

gruppe;

A ein Rest $-CSNH_2$;
oder ein heterocyclischer Rest, ausgewählt aus der Gruppe Oxazol-2-yl, Oxazol-5-yl, Isoxazol-3-yl, Pyrazol-3-yl, Pyrazol-5-yl, Thiazol-2-yl, wobei diese Ringsysteme ein bis zwei Halogenatome und/oder ein bis zwei der folgenden Substituenten tragen können:
- $C_1$-$C_6$-Alkyl, $C_1$-$C_6$-Alkoxy, $C_1$-$C_6$-Alkylthio, welche ihrerseits ein- bis fünffach durch Halogen und/oder einfach durch $C_1$-$C_4$-Alkoxy, $C_1$-$C_4$-Halogenalkoxy, $C_1$-$C_4$-Alkylthio, Amino, $C_1$-$C_4$-Alkylamino, oder Di-$C_1$-$C_4$-alkylamino substituiert sein können;
- $C_3$-$C_6$-Cycloalkyl, Phenyl, welche ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein können;
- ein Rest $-NR^4R^5$ oder
- ein Rest $-COR^6$;

$R^4$, $R^5$ Wasserstoff, Formyl, $C_1$-$C_6$-Alkyl, $C_1$-$C_6$-Halogenalkyl, $C_1$-$C_4$-Alkoxy, $C_1$-$C_4$-Halogenalkoxy, $C_1$-$C_6$-Alkylcarbonyl, welches im Alkylteil ein- bis dreifach durch Halogen substituiert sein kann, $C_1$-$C_6$-Alkoxycarbonyl und/oder Phenylcarbonyl, welches seinerseits ein- bis fünffach durch Halogen und/oder ein- bis dreifach durch $C_1$-$C_4$-Alkyl $C_1$-$C_4$-Halogenalkyl, $C_1$-$C_4$-Alkoxy oder $C_1$-$C_4$-Halogenalkoxy substituiert sein kann,

$R^6$ Hydroxy, $C_1$-$C_6$-Alkoxy, $C_1$-$C_4$-Alkoxy-$C_1$-$C_4$-alkoxy, $C_3$-$C_6$-Alkenyloxy, $C_3$-$C_6$-Alkinyloxy, Phenoxy oder Benzyloxy, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiert sein können, oder eine Gruppe $-NR^7R^8$,

$R^7$, $R^8$ Wasserstoff, $C_1$-$C_6$-Alkyl, $C_3$-$C_6$-Alkenyl, $C_3$-$C_6$-Alkinyl, $C_1$-$C_4$-Alkoxy-$C_1$-$C_4$-alkyl, Phenyl oder Benzyl.

**7.** Verfahren zur Bekämpfung unerwünschten Pflanzenwuchses, dadurch gekennzeichnet, daß man die unerwünschten Pflanzen und/oder ihren Lebensraum mit eine herbizid wirksamen Menge eines Bithienylderivats der allgemeinen Formel Ia gemäß Anspruch 1 behandelt.

**8.** Verwendung von Bithienylderivaten der Formel Ia gemäß Anspruch 1 als herbizide Mittel.

**Claims**
**Claims for the following Contracting States : CH, DE, FR, GB, IT, LI, NL**

**1.** A bithienyl derivative of the general formula Ia

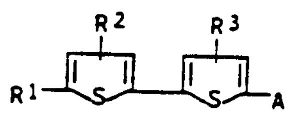

Ia

where
$R^1$ and $R^2$ are each hydrogen, halogen, nitro, formyl, $C_1$-$C_6$-alkyl, $C_1$-$C_6$-haloalkyl, $C_3$-$C_8$-cycloalkyl, $C_3$-$C_8$-cycloalkoxy, $C_1$-$C_6$-alkoxy, $C_1$-$C_4$-haloalkoxy, $C_1$-$C_4$-alkylthio or $C_1$-$C_4$-haloalkylthio, or are each $C_1$-$C_6$-alkylcarbonyl which may be monosubstituted, disubstituted or trisubstituted by halogen, or are each $C_3$-$C_6$-cycloalkylcarbonyl which may be monosubstituted, disubstituted or trisubstituted by halogen and/or by $C_1$-$C_4$-alkyl, or are each $C_1$-$C_6$-alkoxycarbonyl and/or phenylcarbonyl;
$R^3$ is hydrogen, halogen, $C_1$-$C_4$-alkyl, $C_1$-$C_4$-alkoxy, $C_1$-$C_4$-alkylthio, $C_1$-$C_4$-haloalkyl or $C_1$-$C_4$-haloalkoxy;
A is $-CSNH_2$;
or a heterocyclic radical selected from the group consisting of oxazol-2-yl, oxazol-5-yl, isoxazol-3-yl, pyrazol-3-yl, pyrazol-5-yl, thiazol-2-yl, where these ring systems may carry one or two halogen atoms and/or one or two of the following substituents:
$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;
$C_3$-$C_6$-cycloalkyl or phenyl, each of which in turn may be monosubstituted to pentasubstituted by

halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
-$NR^4R^5$ or
-$COR^6$;
$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or $C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl, which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;
$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or is -$NR^7R^8$, and
$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl.

**2.** A process for the preparation of a compound Ia as claimed in claim 1, in which A is -$CSNH_2$, wherein a bithienyl cyanide II

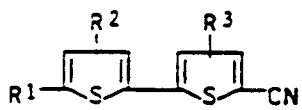

is reacted in a conventional manner
a) with hydrogen sulfide in the presence of a base or
b) with a thiocarboxylic acid III

RCOSH III

where R is lower alkyl, such as methyl, ethyl or 1-methylethyl, in the presence of an acid.

**3.** A process for the preparation of a compound Ia as claimed in claim 1, in which A is oxazol-2-yl or thiazol-2-yl, wherein a bithienylcarboxamide IVa or a bithienylthiocarboxamide Ia (A = $CSNH_2$)

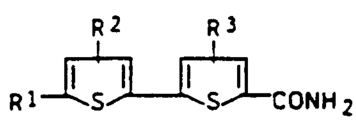

IVa

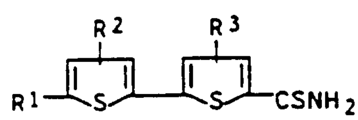

Ia

is reacted in a conventional manner in an inert organic solvent with an α-halocarbonyl compound of the formula V

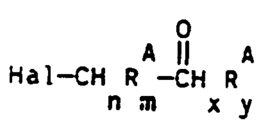

V

where Hal is halogen and $R^A$ is any substituent of the radical A corresponding to claim 1, n is 1 or 2 and n + m is 2, x is 0 or 1 and x + y is 1, or with a corresponding acetal or ketal.

**4.** A process for the preparation of a compound Ia as claimed in claim 1, in which A is oxazol-5-yl, wherein a bithienyl aldehyde derivative VI

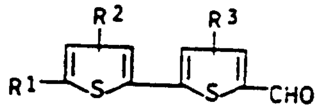

VI

is reacted in a conventional manner in an inert organic solvent in the presence of a base with a corresponding isonitrile of the formula VIIa or VIIb

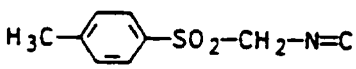

VIIa

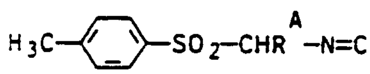

VIIb

where $R^A$ is any substituent of the radical A corresponding to claim 1.

5. A process for the preparation of a compound Ia as claimed in claim 1, in which A is isoxazol-3-yl, wherein a bithienyl nitrile oxide VIII

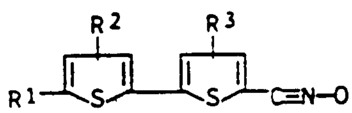

VIII

is reacted in a conventional manner in an inert organic solvent with acetylene or with a corresponding alkyne of the formula IXa or IXb

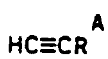

IXa

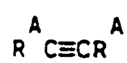

IXb

where $R^A$ has the meaning stated in claim 3.

6. A process for the preparation of a compound Ia as claimed in claim 1, in which A is pyrazol-5-yl, wherein a bithienyldicarbonyl derivative X

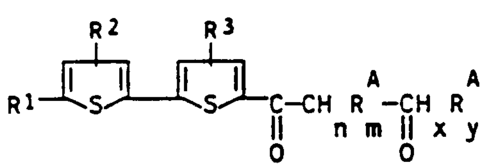

X

wherein n, m, x, y and $R^A$ have the meanings stated in claim 3, is reacted in a conventional manner in an inert organic solvent with hydrazine or with a hydrazine derivative of the formula XI

$H_2N\text{-}NHR^A$ XI.

7. A herbicide containing a bithienyl derivative of the general formula Ia as claimed in claim 1 and conventional inert additives.

8. A method for controlling undesirable plant growth, wherein the undesirable plants and/or their habitat are treated with a herbicidal amount of a bithienyl derivative of the general formula Ia as claimed in claim 1.

9. Use of a bithienyl derivative of the formula Ia as claimed in claim 1 as a herbicide.

**Claims for the following Contracting State : ES**

1. A process for the preparation of a bithienyl derivative of the general formula Ia

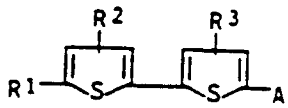

Ia

where

$R^1$ and $R^2$ are each hydrogen, halogen, nitro, formyl, $C_1$-$C_6$-alkyl, $C_1$-$C_6$-haloalkyl, $C_3$-$C_8$-cycloalkyl, $C_3$-$C_8$-cycloalkoxy, $C_1$-$C_6$-alkoxy, $C_1$-$C_4$-haloalkoxy, $C_1$-$C_4$-alkylthio or $C_1$-$C_4$-haloalkylthio, or are each $C_1$-$C_6$-alkylcarbonyl which may be monosubstituted, disubstituted or trisubstituted by halogen, or are each $C_3$-$C_6$-cycloalkylcarbonyl which may be monosubstituted, disubstituted or trisubstituted by halogen and/or by $C_1$-$C_4$-alkyl, or are each $C_1$-$C_6$-alkoxycarbonyl and/or phenylcarbonyl;

$R^3$ is hydrogen, halogen, $C_1$-$C_4$-alkyl, $C_1$-$C_4$-alkoxy, $C_1$-$C_4$-alkylthio, $C_1$-$C_4$-haloalkyl or $C_1$-$C_4$-haloalkoxy;

A is $-CSNH_2$;

wherein a bithienyl cyanide II

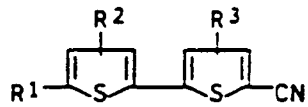

is reacted in a conventional manner

a) with hydrogen sulfide in the presence of a base or

b) with a thiocarboxylic acid III

RCOSH

where R is lower alkyl, such as methyl, ethyl or 1-methylethyl, in the presence of an acid.

2. A process for the preparation of a compound Ia as claimed in claim 1, in which A is a heterocyclic radical selected from the group consisting of thiazol-2-yl, oxazol-2-yl, oxazol-5-yl, isoxazol-3-yl, pyrazol-3-yl, pyrazol-5-yl and thiazol-2-yl, where these ring systems may carry one or two halogen atoms and/or one or two of the following substituents:

$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;

$C_3$-$C_6$-cycloalkyl or phenyl, each of which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;

$-NR^4R^5$ or

$-COR^6$;

$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or $C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl, which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;

$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or is $-NR^7R^8$, and

$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl,
wherein a bithienylcarboxamide IVa or a bithienylthiocarboxamide Ia (A = $CSNH_2$)

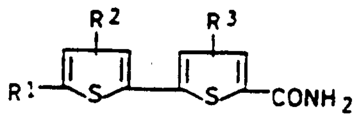
IVa

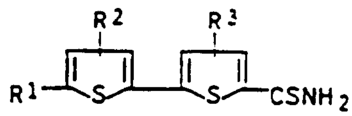
Ia.

is reacted in a conventional manner in an inert organic solvent with an α-halocarbonyl compound of the formula V

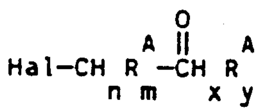
V

where Hal is halogen and $R^A$ is any substituent of the radical A corresponding to claim 1, n is 1 or 2 and n + m is 2, x is 0 or 1 and x + y is 1, or with a corresponding acetal or ketal.

**3.** A process for the preparation of a compound Ia as claimed in claim 1, in which A is a heterocyclic radical selected from the group consisting of oxazol-2-yl, oxazol-5-yl, isoxazol-3-yl, pyrazol-3-yl, pyrazol-5-yl and thiazol-2-yl, where these ring systems may carry one or two halogen atoms and/or one or two of the following substituents:
$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;
$C_3$-$C_6$-cycloalkyl or phenyl, each of which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
$-NR^4R^5$ or
$-COR^6$;
$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or $C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;
$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or is $-NR^7R^8$, and
$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl,
wherein a bithienyl aldehyde derivative VI

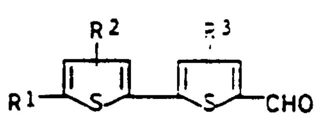
VI

is reacted in a conventional manner in an inert organic solvent in the presence of a base with a corresponding isonitrile of the formula VIIa or VIIb

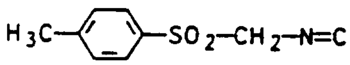

VIIa

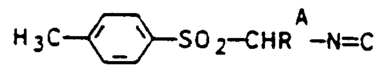

VIIb

where $R^A$ is any substituent of the radical A corresponding to claim 1.

**4.** A process for the preparation of a compound Ia as claimed in claim 1, in which A is a heterocyclic radical selected from the group consisting of oxazol-2-yl, oxazol-5-yl, isoxazol-3-yl, pyrazol-3-yl, pyrazol-5-yl and thiazol-2-yl, where these ring systems may carry one or two halogen atoms and/or one or two of the following substituents:
$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;
$C_3$-$C_6$-cycloalkyl or phenyl, each of which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
$-NR^4R^5$ or
$-COR^6$;
$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or $C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;
$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or is $-NR^7R^8$, and
$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl,
wherein a bithienylnitrile oxide VIII

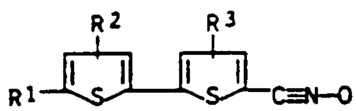

VIII

is reacted in a conventional manner in an inert organic solvent with acetylene or with a corresponding alkyne of the formula IXa or IXb

$HC\equiv CR^A$

IXa

$R^A C\equiv CR^A$

IXb

where $R^A$ has the meanings stated in claim 2.

**5.** A process for the preparation of a compound Ia as claimed in claim 1, in which A is a heterocyclic radical selected from the group consisting of oxazol-2-yl, oxazol-5-yl, isoxazol-3-yl, pyrazol-3-yl, pyrazol-5-yl and thiazol-2-yl, where these ring systems may carry one or two halogen atoms and/or one or two of the following substituents:
$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;
$C_3$-$C_6$-cycloalkyl or phenyl, each of which in turn may be monosubstituted to pentasubstituted by

halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
-$NR^4R^5$ or
-$COR^6$;
$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or $C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;
$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or -is $NR^7R^8$, and
$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl, wherein a bithienyldicarbonyl derivative X

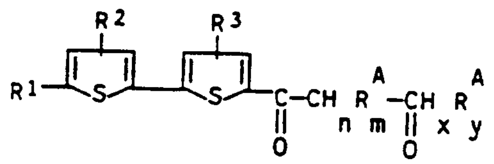

X

where n, m, x, y and $R^A$ have the meanings stated in claim 2, is reacted in a conventional manner in an inert organic solvent with hydrazine or with a hydrazine derivative of the formula XI

$H_2N$-$NHR^A$ XI.

**6.** A herbicide containing conventional inert additives and a bithienyl derivative of the general formula Ia

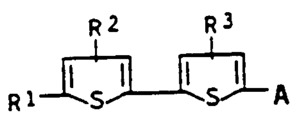

Ia

where
$R^1$ and $R^2$ are each hydrogen, halogen, nitro, formyl, $C_1$-$C_6$-alkyl, $C_1$-$C_6$-haloalkyl, $C_3$-$C_8$-cycloalkyl, $C_3$-$C_8$-cycloalkoxy, $C_1$-$C_6$-alkoxy, $C_1$-$C_4$-haloalkoxy, $C_1$-$C_4$-alkylthio or $C_1$-$C_4$-haloalkylthio, or are each $C_1$-$C_6$-alkylcarbonyl which may be monosubstituted, disubstituted or trisubstituted by halogen, or are each $C_3$-$C_6$-cycloalkylcarbonyl which may be monosubstituted, disubstituted or trisubstituted by halogen and/or by $C_1$-$C_4$-alkyl, or are each $C_1$-$C_6$-alkoxycarbonyl and/or phenylcarbonyl;
$R^3$ is hydrogen, halogen, $C_1$-$C_4$-alkyl, $C_1$-$C_4$-alkoxy, $C_1$-$C_4$-alkylthio, $C_1$-$C_4$-haloalkyl or $C_1$-$C_4$-haloalkoxy;
A is -$CSNH_2$;
or a heterocyclic radical selected from the group consisting of oxazol-2-yl, oxazol-5-yl, isoxazol-3-yl, pyrazol-3-yl, pyrazol-5-yl and thiazol-2-yl, where these ring systems may carry one or two halogen atoms and/or one or two of the following substituents:
$C_1$-$C_6$-alkyl, $C_1$-$C_6$-alkoxy or $C_1$-$C_6$-alkylthio, each of which in turn may be monosubstituted to pentasubstituted by halogen and/or monosubstituted by $C_1$-$C_4$-alkoxy, $C_1$-$C_4$-haloalkoxy, $C_1$-$C_4$-alkylthio, amino, $C_1$-$C_4$-alkylamino or di-$C_1$-$C_4$-alkylamino;
$C_3$-$C_6$-cycloalkyl or phenyl, each of which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
-$NR^4R^5$ or
-$COR^6$;
$R^4$ and $R^5$ are each hydrogen, formyl, $C_1$-$C_6$-alkyl, $C_1$-$C_6$-haloalkyl, $C_1$-$C_4$-alkoxy, $C_1$-$C_4$-haloalkoxy or

$C_1$-$C_6$-alkylcarbonyl which may be monosubstituted, disubstituted or trisubstituted in the alkyl moiety by halogen, or are each $C_1$-$C_6$-alkoxycarbonyl and/or phenylcarbonyl which in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy;

$R^6$ is hydroxyl, $C_1$-$C_6$-alkoxy, $C_1$-$C_4$-alkoxy-$C_1$-$C_4$-alkoxy, $C_3$-$C_6$-alkenyloxy, $C_3$-$C_6$-alkynyloxy, phenoxy or benzyloxy, where the aromatic rings in turn may be monosubstituted to pentasubstituted by halogen and/or monosubstituted, disubstituted or trisubstituted by $C_1$-$C_4$-alkyl, $C_1$-$C_4$-haloalkyl, $C_1$-$C_4$-alkoxy or $C_1$-$C_4$-haloalkoxy, or is $-NR^7R^8$, and

$R^7$ and $R^8$ are each hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl, phenyl or benzyl.

**7.** A method for controlling undesirable plant growth, wherein the undesirable plants and/or their habitat are treated with a herbicidal amount of a bithienyl derivative of the general formula Ia as claimed in claim 1.

**8.** Use of a bithienyl derivative of the formula Ia as claimed in claim 1 as a herbicide.

**Revendications**
**Revendications pour les Etats contractants suivants : CH, DE, FR, GB, IT, LI, NL**

**1.** Dérivés bithiényliques de formule générale Ia

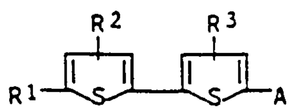

Ia

dans laquelle les symboles ont les significations suivantes :

$R^1$, $R^2$ : l'hydrogène, un halogène, un groupe nitro, formyle, alkyle en $C_1$-$C_6$, halogénoalkyle en $C_1$-$C_6$, cycloalkyle en $C_3$-$C_8$, cycloalcoxy en $C_3$-$C_8$, alcoxy en $C_1$-$C_6$, halogénoalcoxy en $C_1$-$C_4$, alkylthio en $C_1$-$C_4$, halogénoalkylthio en $C_1$-$C_4$, (alkyle en $C_1$-$C_6$)-carbonyle, lequel peut être substitué par 1 à 3 atomes d'halogènes, (cycloalkyle en $C_3$-$C_6$)-carbonyle, lequel peut porter un à trois substituants halogéno et/ou alkyle en $C_1$-$C_4$, (alcoxy en $C_1$-$C_6$)-carbonyle et/ou phénylcarbonyle ;

$R^3$ : l'hydrogène, un atome d'halogène, un groupe alkyle en $C_1$-$C_4$, un groupe alcoxy en $C_1$-$C_4$, un groupe alkylthio en $C_1$-$C_4$, un groupe halogénoalkyle en $C_1$-$C_4$ ou un groupe halogénoalcoxy en $C_1$-$C_4$ ;

A : un groupe $-CSNH_2$ ;

ou bien un radical hétérocyclique choisi parmi les radicaux oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 des substituants suivants :

- alkyle en $C_1$-$C_6$, alcoxy en $C_1$-$C_6$, alkylthio en $C_1$-$C_6$, qui peuvent porter 1 à 5 substituants halogéno et/ou un substituant alcoxy en $C_1$-$C_4$, halogénoalcoxy en $C_1$-$C_4$, alkylthio en $C_1$-$C_4$, amino, alkylamino en $C_1$-$C_4$ ou di-(alkyle en $C_1$-$C_4$)-amino ;
- cycloalkyle en $C_3$-$C_6$, phényle, qui peuvent eux-mêmes porter 1 à 5 substituants halogéno et/ou 1 à 3 substituants alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
- un groupe $-NH_4R^5$ ou bien
- un groupe $-COR^6$ ;

$R^4$, $R^5$ : l'hydrogène, un groupe formyle, alkyle en $C_1$-$C_6$,halogénoalkyle en $C_1$-$C_6$, alcoxy en $C_1$-$C_4$, halogénoalcoxy en $C_1$-$C_4$, (alkyle en $C_1$-$C_6$)-carbonyle, lequel peut être substitué dans la partie alkyle par 1 à 3 atomes d'halogènes, (alcoxy en $C_1$-$C_6$)-carbonyle et/ou phénylcarbonyle, lequel peut lui-même être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$ ;

$R^6$ : un groupe hydroxy, alcoxy en $C_1$-$C_6$, (alcoxy en $C_1$-$C_4$)-alcoxy en $C_1$-$C_4$, alcényloxy en $C_3$-$C_6$, alcynyloxy en $C_3$-$C_6$, phénoxy ou benzyloxy, les noyaux aromatiques pouvant

eux-mêmes porter 1 à 5 substituants halogéno et/ou 1 à 3 substituants alkyle en $C_1$-$C_4$, halogénoalkyle en $C_1$-$C_4$, alcoxy en $C_1$-$C_4$ ou halogénoalcoxy en $C_1$-$C_4$, ou un groupe $-NR^7R^8$,

$R^7$, $R^8$ : l'hydrogène, un groupe alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle.

**2.** Procédé de préparation des composés Ia de la revendication 1 pour lesquels A représente un groupe $-CSNH_2$, caractérisé en ce que l'on fait réagir un cyanure de bithiényle II

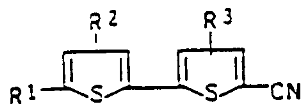

de manière connue en soi

a) avec le sulfure d'hydrogène en présence d'une base, ou bien

b) avec un acide thiocarboxylique de formule III

RCOSH III

dans laquelle R représente un groupe alkyle inférieur tel que méthyle, éthyle ou 1-méthyléthyle, en présence d'un acide.

**3.** Procédé de préparation des composés Ia de la revendication 1 pour lesquels A représente un groupe oxazole-2-yle ou thiazole-2-yle, caractérisé en ce que l'on fait réagir un bithiénylcarboxamide IVa ou respectivement un bithénylthiocarboxamide Ia (A = $CSNH_2$)

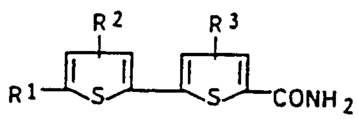

IVa

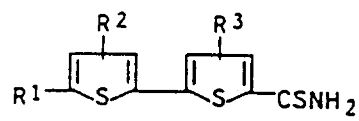

Ia

de manière connue en soi, dans un solvant organique inerte, avec un dérivé α-halogéno-carbonylé de formule V

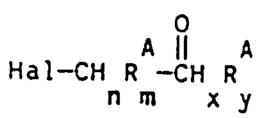

V

dans laquelle Hal représente un atome d'halogène et $R^A$ représente un substituant quelconque du groupe A tel que défini dans la revendication 1, n est égal à 1 ou 2 et la somme n + m est égale à 2, x est égal à 0 ou 1, et la somme x + y est égale à 1, ou avec un acétal correspondant.

**4.** Procédé de préparation des composés Ia de la revendication 1 pour lesquels A représente un groupe oxazole-5-yle, caractérisé en ce que l'on fait réagir un aldéhyde bithiénylique VI

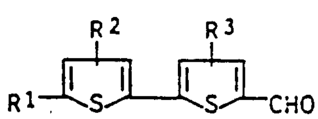

VI

de manière connue en soi, dans un solvant organique inerte, en présence d'une base, avec un isonitrile correspondant de formule respective VIIa ou VIIb

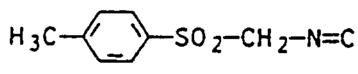

VIIa

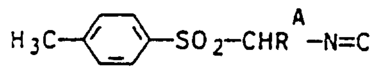

VIIb

dans laquelle $R^A$ représente un substituant quelconque du groupe A de la revendication 1.

**5.** Procédé de préparation des composés Ia de la revendication 1 pour lesquels A représente un groupe isoxazole-3-yle, caractérisé en ce que l'on fait réagir un oxyde de bithiénylnitrile VIII

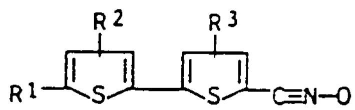

VIII

de manière connue en soi, dans un solvant organique inerte, avec l'acétylène ou un alcyne correspondant de formules respectives IXa et IXb

$HC\equiv CR^A$

IXa

$R^A C\equiv CR^A$

IXb

dans lesquelles $R^A$ a les significations indiquées dans la revendication 3.

**6.** Procédé de préparation des composés Ia de la revendication 1 pour lesquels A représente un groupe pyrazole-5-yle, caractérisé en ce que l'on fait réagir un dérivé bithiényldicarbonylé de formule X

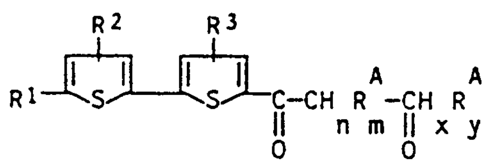

X

dans laquelle n, m, x, y et $R^A$ ont les signfications indiquées dans la revendication 3, de manière connue en soi, dans un solvant organique inerte, avec l'hydrazine ou un dérivé de l'hydrazine de formule XI

$H_2N\text{-}NHR^A$  XI

**7.** Produits herbicides contenant un dérivé bithiénylique de formule générale Ia de la revendication 1 et des additifs inertes usuels.

**8.** Procédé pour combattre les croissances de végétaux indésirables, caractérisé en ce que l'on traite les végétaux indésirables et/ou leur habitat par une quantité herbicide efficace d'un dérivé bithiénylique de formule générale Ia de la revendication 1.

**9.** Utilisation des dérivés bithiényliques de formule Ia de la revendication 1 en tant que produits herbicides.

**Revendications pour l'Etat contractant suivant : ES**

1. Procédé de préparation des dérivés bithiényliques de formule générale Ia

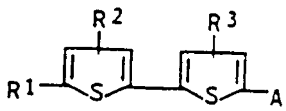

I a

dans laquelle les symboles ont les signfications suivantes :

$R^1$, $R^2$ : l'hydrogène, un halogène, un groupe nitro, formyle, alkyle en $C_1$-$C_6$, halogénoalkyle en $C_1$-$C_6$, cycloalkyle en $C_3$-$C_8$, cycloalcoxy en $C_3$-$C_8$, alcoxy en $C_1$-$C_6$, halogénoalcoxy en $C_1$-$C_4$, alkylthio en $C_1$-$C_4$, halogénoalkylthio en $C_1$-$C_4$, (alkyle en $C_1$-$C_6$)-carbonyle, lequel peut être substitué par 1 à 3 atomes d'halogènes, (cycloalkyle en $C_3$-$C_6$)-carbonyle, lequel peut porter un à trois substituants halogéno et/ou alkyle en $C_1$-$C_4$, (alcoxy en $C_1$-$C_6$)-carbonyle et/ou phénylcarbonyle ;

$R^3$ : l'hydrogène, un atome d'halogène, un groupe alkyle en $C_1$-$C_4$, un groupe alcoxy en $C_1$-$C^4$, un groupe alkylthio en $C_1$-$C_4$, un groupe halogénoalkyle en $C_1$-$C_4$ ou un groupe halogénoalcoxy en $C_1$-$C_4$ ;

A : un groupe $-CSNH_2$ ;

caractérisé en ce que l'on fait réagir un cyanure de bithiényle II

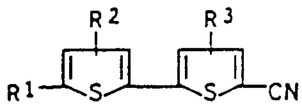

de manière connue en soi,

a) avec le sulfure d'hydrogène en présence d'une base, ou bien

b) avec un acide thiocarboxylique III

RCOSH III

pour lequel R représente un groupe alkyle inférieur tel que méthyle, éthyle ou 1-méthyléthyle, en présence d'un acide.

2. Procédé de préparation des composés Ia de la revendication pour lesquels A représente un groupe hétérocyclique choisi parmi les groupes thiazole-2-yle, oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 substituants suivants :
   - alkyle en $C_1$-$C_6$, alcoxy en $C_1$-$C_6$, alkylthio en $C_1$-$C_6$, qui peuvent eux-mêmes porter 1 à 5 substituants halogéno et/ou un substituant alcoxy en $C_1$-$C_4$, halogénoalcoxy en $C_1$-$C_4$, alkylthio en $C_1$-$C_4$, amino, alkylamino en $C_1$-$C_4$, ou di-(alkyle en $C_1$-$C_4$)-amino ;
   - cycloalkyle en $C_3$-$C_6$, phényle, qui peuvent eux-mêmes porter 1 à 5 substituants halogéno et/ou 1 à 3 substituants alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
   - un groupe $-NR^4R^5$ ou bien
   - un groupe $-COR^6$ ;

   $R^4$, $R^5$ : l'hydrogène, un groupe formyle, alkyle en $C_1$-$C_6$,halogénoalkyle en $C_1$-$C_6$, alcoxy en $C_1$-$C_4$, halogénoalcoxy en $C_1$-$C_4$, (alkyle en $C_1$-$C_6$)-carbonyle, lequel peut être substitué dans la partie alkyle par 1 à 3 atomes d'halogènes, (alcoxy en $C_1$-$C_6$)-carbonyle et/ou phénylcarbonyle, lequel peut être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$,

   $R^6$ : un groupe hydroxy, alcoxy en $C_1$-$C_6$, (alcoxy en $C_1$-$C_4$)-alcoxy en $C_1$-$C_4$, alcényloxy en $C_3$-$C_6$, alcynyloxy en $C_3$-$C_6$, phénoxy ou benzyloxy, les noyaux aromatiques pouvant

eux-mêmes porter 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$, ou un groupe -$NR^7R^8$,

$R^7$, $R^8$ : l'hydrogène, un groupe alkyle alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle,

caractérisé en ce que l'on fait réagir un bithiénylcarboxamide IVa ou un bithiénylthiocarboxamide Ia (A = $CSNH_2$)

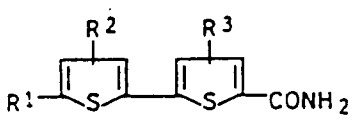

IVa

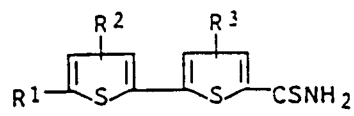

Ia

de manière connue en soi, dans un solvant organique inerte, avec un dérivé α-halogénocarbonylé de formule V

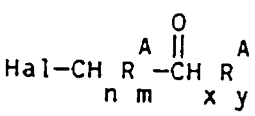

V

dans laquelle Hal représente un atome d'halogène et $R^A$ un substituant quelconque du groupe A tel que défini dans la revendication 1, n est égal à 1 ou 2 et la somme n + m est égale à 2, x est égal à 0 ou 1 et la somme x + y est égale à 1, ou avec un acétal correspondant.

3. Procédé de préparation des composés Ia de la revendication 1 pour lesquels A représente un groupe hétérocyclique choisi parmi les groupes oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 des substituants suivants :

- alkyle en $C_1$-$C_6$, alcoxy en $C_1$-$C_6$, alkylthio en $C_1$-$C_6$, qui peuvent eux-mêmes être substitués par 1 à 5 atomes d'halogènes et/ou un groupe alcoxy en $C_1$-$C_4$, halogénoalcoxy en $C_1$-$C_4$, alkylthio en $C_1$-$C_4$,amino, alkylamino en $C_1$-$C_4$ ou di-(alkyle en $C_1$-$C_4$)-amino ;
- cycloalkyle en $C_3$-$C_6$, phényle, qui peuvent eux-mêmes porter 1 à 5 substituants halogéno et/ou 1 à 3 substituants alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
- un groupe -$NR^4R^5$, ou bien
- un groupe -$COR^6$ ;

$R^4$, $R^5$ : l'hydrogène, un groupe formyle, alkyle en $C_1$-$C_6$,haloégénoalkyle en $C_1$-$C_6$, alcoxy en $C_1$-$C_4$, halogénoalcoxy en $C_1$-$C_4$, (alkyle en $C_1$-$C_6$)-carbonyle, lequel peut être substitué dans la partie alkyle par 1 à 3 atomes d'halogènes, (alcoxy en $C_1$-$C_6$)-carbonyle et/ou phénylcarbonyle, lequel peut être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$ ;

$R^6$ : un groupe hydroxy, alcoxy en $C_1$-$C_6$ (alcoxy en $C_1$-$C_4$)-alcoxy en $C_1$-$C_4$, alcényloxy en $C_3$-$C_6$, alcynyloxy en $C_3$-$C_6$, phénoxy ou benzyloxy, les noyaux aromatiques pouvant eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$, ou un groupe -$NR^7R^8$,

$R^7$, $R^8$ : l'hydrogène, un groupe alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle,

caractérisé en ce que l'on fait réagir un aldéhyde bithiénylique

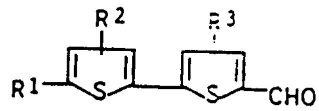

VI

de manière connue en soi, dans un solvant organique inerte, en présence d'une base, avec un isonitrile correspondant de formule respective VIIa ou VIIb

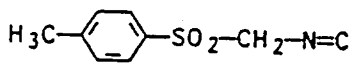

VIIa

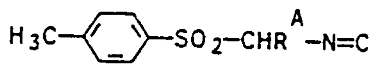

VIIb

dans laquelle $R^A$ représente un substituant quelconque du groupe A tel que défini dans la revendication 1.

**4.** Procédé de préparation des composés Ia de la revendication 1, pour lesquels A représente un groupe hétérocyclique choisi parmi les groupes oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 des substituants suivants :

- alkyle en $C_1$-$C_6$, alcoxy en $C_1$-$C_6$, alkylthio en $C_1$-$C_6$, qui peuvent eux-mêmes être substitués par 1 à 5 atomes d'halogènes et/ou par un groupe alcoxy en $C_1$-$C_4$, halogénoalcoxy en $C_1$-$C_4$, alkylthio en $C_1$-$C_4$,amino, alkylamino en $C_1$-$C_4$, ou di-(alkyle en $C_1$-$C_4$)-amino ;
- cycloalkyle en $C_3$-$C_6$, phényle, qui peuvent eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
- un groupe $-NR^4R^5$, ou bien
- un groupe $-COR^6$ ;

R4, R5 : l'hydrogène, un groupe formyle, alkyle en $C_1$-$C_6$,halogénoalkyle en $C_1$-$C_6$, alcoxy en $C_1$-$C_4$, halogénoalcoxy en $C_1$-$C_4$, (alkyle en $C_1$-$C_6$)-carbonyle, lequel peut lui-même être substitué dans la partie alkyle par 1 à 3 atomes d'halogènes, (alcoxy en $C_1$-$C_6$)-carbonyle et/ou phénylcarbonyle, lequel peut lui-même être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$,

$R^5$ : un groupe hydroxy, alcoxy en $C_1$-$C_6$, (alcoxy en $C_1$-$C_4$)-alcoxy en $C_1$-$C_4$, alcényloxy en $C_3$-$C_6$, alcynyloxy en $C_3$-$C_6$, phénoxy ou benzyloxy, les noyaux aromatiques pouvant eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$, ou un groupe $-NR^7R^8$,

$R^7$, $R^8$ : l'hydrogène, un groupe alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle,

caractérisé en ce que l'on fait réagir un oxyde de bithiénylnitrile VIII

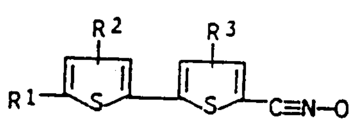

VIII

de manière connue en soi, dans un solvant organique inerte, avec l'acétylène ou un alcyne correspondant de formule respective IXa ou IXb

$HC\equiv CR^A$      $R^AC\equiv CR^A$

IXa      IXb

dans lesquelles $R^A$ a les significations indiquées dans la revendication 2.

**5.** Procédé de préparation des composés Ia de la revendication 1 pour lesquels A représente un groupe hétérocyclique choisi parmi les groupes oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 des substituants suivants :

- alkyle en $C_1$-$C_6$, alcoxy en $C_1$-$C_6$, alkylthio en $C_1$-$C_6$, qui peuvent eux-mêmes être substitués par 1 à 5 atomes d'halogènes et/ou un groupe alcoxy en $C_1$-$C_4$, halogénoalcoxy en $C_1$-$C_4$, alkylthio en $C_1$-$C_4$,amino, alkylamino en $C_1$-$C_4$ ou di-(alkyle en $C_1$-$C_4$)-amino ;
- cycloalkyle en $C_3$-$C_6$, phényle, qui peuvent eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
- un groupe $-NR^4R^5$, ou bien
- un groupe $-COR^6$ ;

$R^4$, $R^5$ : l'hydrogène, un groupe formyle, alkyle en $C_1$-$C_6$,halogénoalkyle en $C_1$-$C_6$, alcoxy en $C_1$-$C_4$, halogénoalcoxy en $C_1$-$C_4$, (alkyle en $C_1$-$C_6$)-carbonyle, lequel peut être substitué dans la partie alkyle par 1 à 3 atomes d'halogènes, (alcoxy en $C_1$-$C_6$)-carbonyle et/ou phénylcarbonyle, lequel peut être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$,

$R^6$ : un groupe hydroxy, alcoxy en $C_1$-$C_6$, (alcoxy en $C_1$-$C_4$)-alcoxy en $C_1$-$C_4$, alcényloxy en $C_3$-$C_6$, alcynyloxy en $C_3$-$C_6$, phénoxy ou benzyloxy, les noyaux aromatiques pouvant eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$ ou halogénoalcoxy en $C_1$-$C_4$, ou un groupe $-NR^7R^8$,

$R^7$, $R^8$: l'hydrogène, un groupe alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle,

caractérisé en ce que l'on fait réagir un dérivé bithiényldicarbonylé de formule X

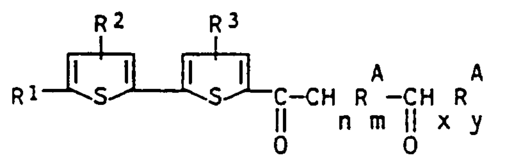

X

dans laquelle n, m, x, y et $R^A$ ont les signfications indiquées dans la revendication 2, de manière connue en soi, dans un solvant organique inerte, avec l'hydrazine ou un dérivé de l'hydrazine de formule XI

$H_2N$-$NHR^A$     XI

**6.** Produits herbicides contenant des additifs inertes usuels et un dérivé bithiénylique de formule générale Ia

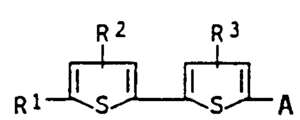

Ia

dans laquelle les symboles ont les signfications suivantes :

$R^1$, $R^2$ : l'hydrogène, un halogène, un groupe nitro, formyle, alkyle en $C_1$-$C_6$, halogénoalkyle en $C_1$-$C_6$, cycloalkyle en $C_3$-$C_8$, cycloalcoxy en $C_3$-$C_8$, alcoxy en $C_1$-$C_6$, halogénoalcoxy en $C_1$-$C_4$, alkylthio en $C_1$-$C_4$, halogénoalkylthio en $C_1$-$C_4$, (alkyle en $C_1$-$C_6$)-carbonyle, lequel peut êter substitué par 1 à 3 atomes d'halogènes, (cycloalkyle en $C_3$-$C_6$)-carbonyle, lequel peut être substitué par 1 à 3 atomes d'halogènes et/ou groupes alkyle en $C_1$-$C_4$, (alcoxy en $C_1$-$C_6$)-carbonyle et/ou phénylcarbonyle ;

$R^3$ : l'hydrogène, un atome d'halogène, un groupe alkyle en $C_1$-$C_4$, un groupe alcoxy en $C_1$-$C_4$, un groupe alkylthio en $C_1$-$C_4$, un groupe halogénoalkyle en $C_1$-$C_4$, ou un groupe halogénoalcoxy en $C_1$-$C_4$ ;

A : un groupe $-CSNH_2$ ;

ou bien un groupe hétérocyclique choisi parmi les groupes oxazole-2-yle, oxazole-5-yle, isoxazole-3-yle, pyrazole-3-yle, pyrazole-5-yle, thiazole-2-yle, ces systèmes cycliques pouvant porter 1 à 2 atomes d'halogènes et/ou 1 à 2 des substitutants suivants :

- alkyle en $C_1$-$C_6$, alcoxy en $C_1$-$C_6$, alkylthio en $C_1$-$C_6$, qui peuvent eux-mêmes être substitués par 1 à 5 atomes d'halogènes et/ou par un groupe alcoxy en $C_1$-$C_4$, halogénoalcoxy en $C_1$-$C_4$, alkylthio en $C_1$-$C_4$,amino, alkylamino en $C_1$-$C_4$ ou di-(alkyle en $C_1$-$C_4$)-amino ;
- cycloalkyle en $C_3$-$C_6$, phényle, qui peuvent eux-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
- un groupe $-NR^4R^5$ ou bien
- un groupe $-COR^6$ ;

$R^4$, $R^5$ : l'hydrogène, un groupe formyle, alkyle en $C_1$-$C_6$,halogénoalkyle en $C_1$-$C_6$, alcoxy en $C_1$-$C_4$, halogénoalcoxy en $C_1$-$C_4$, (alkyle en $C_1$-$C_6$)-carbonyle, lequel peut être substitué dans la partie alkyle par 1 à 3 atomes d'halogènes, (alcoxy en $C_1$-$C_6$)-carbonyle et/ou phénylcarbonyle, lequel peut être substitué par 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$,

$R^6$ : un groupe hydroxy, alcoxy en $C_1$-$C_6$, (alcoxy en $C_1$-$C_4$)-alcoxy en $C_1$-$C_4$, alcényle en $C_3$-$C_6$, alcynyloxy en $C_3$-$C_6$, phénoxy ou benzyloxy, les noyaux aromatiques pouvant euxx-mêmes être substitués par 1 à 5 atomes d'halogènes et/ou 1 à 3 groupes alkyle en $C_1$-$C_4$, halogénoalkyle en $C_1$-$C_4$, alcoxy en $C_1$-$C_4$, halogénoalcoxy en $C_1$-$C_4$, ou bien un groupe $-NR^7R^8$,

$R^7$, $R^8$ : l'hydrogène, un groupe alkyle en $C_1$-$C_6$, alcényle en $C_3$-$C_6$, alcynyle en $C_3$-$C_6$, (alcoxy en $C_1$-$C_4$)-alkyle en $C_1$-$C_4$, phényle ou benzyle.

**7.** Procédé pour combattre les croissances de végétaux indésirables, caractérisé en ce que l'on traite les végétaux indésirables et/ou leur habitat par une quantité herbicide efficace d'un dérivé bithiénylique de formule générale Ia de la revendication 1.

**8.** Utilisation des dérivés bithiényliques de formule Ia de la revendication 1 en tant que produits herbicides.